# EUROPEAN PATENT APPLICATION

(11) **EP 3 741 861 A1**
(43) Date of publication of application: **25.11.2020**
(21) Application number: 19741277.8
(22) Date of filing: 21.01.2019
(51) Int. Cl.: C12N 15/85, C12N 15/90, A61P 35/00

(54) **SYNNOTCH RECEPTOR-REGULATED EXPRESSION OF IL12**

(30) Priority: 19.01.2018 CN 201810053219
(71) Applicant: Carsgen Therapeutics Co., Ltd., Shanghai 200231 (CN); Shanghai Cancer Institute, Shanghai 200032 (CN)
(72) Inventor: LI, Zonghai, Shanghai 200231 (CN); LUO, Hong, Shanghai 200231 (CN); WANG, Huamao, Shanghai 200231 (CN)
(74) Representative: reuteler & cie SA
(86) International application number: PCT/CN2019/072497
(87) International publication number: WO 2019/141270

(57) **Abstract**

A binary vector and the provision of expression of a target antigen-dependent regulatory cytokine using the synNotch technology, so as to partially release the cytokine to enhance the function of T cell and reduce the non-specific toxic and side effect of the cytokine. A synthetic notch receptor is used to construct plasmid vectors to achieve local cytokine secretion. One plasmid vector uses a specific antibody as an extracellular domain for identifying a target antigen, a notch core comprises a transmembrane domain and a specific enzyme cutting site on a notch receptor, and transcription factor GAL4VP64 is used as an intracellular domain. When the target antigen is identified, the enzyme cutting site on the notch will be identified by a corresponding enzyme to hydrolyze and cut the intercellular transcription factor GAL4VP64. Another plasmid vector carries DNA identification/binding domain GAL4UAS of transcription factor GAL4 and correspondingly activates a minimal promoter of a target gene, and after being hydrolyzed and cut, GAL4VP64 will be bound to the binding domain to activate transcription of subsequent genes so as to express IL12 and secrete same out of the cell.

SEQUENCE LISTING

<110>  CARSGEN THERAPEUTICS CO., LTD.;
       SHANGHAI CANCER INSTITUTE

<120>  SYNNOTCH RECEPTOR-REGULATED EXPRESSION OF IL12

<130>  P19400153C

<150>  CN201810053219.6
<151>  2018-01-19

<160>  29

<170>  PatentIn version 3.5

<210>  1
<211>  63
<212>  DNA
<213>  Artificial Sequence

<220>
<223>  Signal sequence

<400>  1
atggcgctcc ctgtcaccgc actgcttctt ccgctggcac tgctgctgca cgctgcacgg      60

cct                                                                    63


<210>  2
<211>  729
<212>  DNA
<213>  Artificial Sequence

<220>
<223>  GPC3-scFv

<400>  2
gaggtgcagc tggtgcagag cggcgccgag gtgaagaagc ccggcgccag cgtgaaggtg      60

agctgcaagg ccagcggcta caccttcagc gactacgaga tgcactgggt gcggcaggcc     120

cccggccagg gcctggagtg gatgggcgcc atccaccccg gcagcggcga caccgcctac     180

aaccagcggt tcaagggccg ggtgaccatc accgccgaca agagcaccag caccgcctac     240

atggagctga gcagcctgcg gagcgaggac accgccgtgt actactgcgc ccggttctac     300

agctacgcct actggggcca gggcaccctg gtgaccgtga gcgccggtgg aggcggttca     360

ggcggaggtg gttctggcgg tggcggatcg gacatcgtga tgacccagac ccccctgagc     420

ctgcccgtga cccccggcga gcccgccagc atcagctgcc ggagcagcca gagcctggtg     480

cacagcaacg gcaacaccta cctgcagtgg tacctgcaga agcccggcca gagcccccag     540

ctgctgatct acaaggtgag caaccggttc agcggcgtgc ccgaccggtt cagcggcagc     600

ggcagcggca ccgacttcac cctgaagatc agccgggtgg aggccgagga cgtgggcgtg     660

tactactgca gccagagcat ctacgtgccc tacaccttcg gccagggcac caagctggag     720

atcaaacgt                                                          729


<210>  3
<211>  978
<212>  DNA
<213>  Artificial Sequence

<220>
<223>  notch core

<400>  3
atcctggact acagcttcac aggtggcgct gggcgcgaca ttcccccacc gcagattgag      60

gaggcctgtg agctgcctga gtgccaggtg gatgcaggca ataaggtctg caacctgcag     120

tgtaataatc acgcatgtgg ctgggatggt ggcgactgct ccctcaactt caatgacccc     180

tggaagaact gcacgcagtc tctacagtgc tggaagtatt ttagcgacgg ccactgtgac     240

agccagtgca actcggccgg ctgcctcttt gatggcttcg actgccagct caccgaggga     300

cagtgcaacc ccctgtatga ccagtactgc aaggaccact tcagtgatgg ccactgcgac     360

cagggctgta acagtgccga atgtgagtgg gatggcctag actgtgctga gcatgtaccc     420

gagcggctgg cagccggcac cctggtgctg gtggtgctgc ttccacccga ccagctacgg     480

aacaactcct tccactttct gcgggagctc agccacgtgc tgcacaccaa cgtggtcttc     540

aagcgtgatg cgcaaggcca gcagatgatc ttcccgtact atggccacga ggaagagctg     600

cgcaagcacc caatcaagcg ctctacagtg ggttgggcca cctcttcact gcttcctggt     660

accagtggtg ggcgccagcg cagggagctg gaccccatgg acatccgtgg ctccattgtc     720

tacctggaga tcgacaaccg gcaatgtgtg cagtcatcct cgcagtgctt ccagagtgcc     780

accgatgtgg ctgccttcct aggtgctctt gcgtcacttg gcagcctcaa tattccttac     840

aagattgagg ccgtgaagag tgagccggtg gagcctccgc tgccctcgca gctgcacctc     900

atgtacgtgg cagcggccgc cttcgtgctc ctgttctttg tgggctgtgg ggtgctgctg     960

tcccgcaagc gccggcgg                                                   978


<210>  4
<211>  633
<212>  DNA
<213>  Artificial Sequence

<220>
<223>  Gal4VP64

<400>  4
atgaagctgc tgagcagcat cgagcaggcc tgtgacatct gccggctgaa gaaactgaag      60

tgcagcaaag aaaagcccaa gtgcgccaag tgcctgaaga acaactggga gtgccggtac     120

agccccaaga ccaagagaag ccccctgacc agagcccacc tgaccgaggt ggaaagccgg     180

ctggaaagac tggaacagct gtttctgctg atcttcccac gcgaggacct ggacatgatc     240

ctgaagatgg acagcctgca ggacatcaag gccctgctga ccggcctgtt cgtgcaggac 300

aacgtgaaca aggacgccgt gaccgacaga ctggccagcg tggaaaccga catgcccctg 360

accctgcggc agcacagaat cagcgccacc agcagcagcg aggaaagcag caacaagggc 420

cagcggcagc tgacagtgtc tgctgctgca ggcggaagcg gaggctctgg cggatctgat 480

gccctggacg acttcgacct ggatatgctg ggcagcgacg ccctggatga ttttgatctg 540

gacatgctgg gatctgacgc tctggacgat ttcgatctcg acatgttggg atcagatgca 600

ctggatgact ttgacctgga catgctcgga tca 633

<210> 5
<211> 128
<212> DNA
<213> Artificial Sequence

<220>
<223> GAL4UAS

<400> 5
tcggagcact gtcctccgaa cgtcggagca ctgtcctccg aacgtcggag cactgtcctc 60

cgaacgtcgg agcactgtcc tccgaacgga gcatgtcctc cgaacgtcgg agcactgtcc 120

tccgaacg 128

<210> 6
<211> 144
<212> DNA
<213> Artificial Sequence

<220>
<223> minimal CMV promoter

<400> 6
actagttagg cgtgtacggt gggaggccta tataagcaga gctcgtttag tgaaccgtca 60

gatcgcctgg agacgccatc cacgctgttt tgacctccat agaagacacc gggaccgatc 120

cagcctctcg acattcgttg gatc 144

<210> 7
<211> 918
<212> DNA
<213> Artificial Sequence

<220>
<223> IL12 P40

<400> 7
atatgggaac tgaagaaaga tgtttatgtc gtagaattgg attggtatcc ggatgcccct 60

ggagaaatgg tggtcctcac ctgtgacacc cctgaagaag atggtatcac ctggaccttg 120

gaccagagca gtgaggtctt aggctctggc aaaaccctga ccatccaagt caaagagttt 180

ggagatgctg gccagtacac ctgtcacaaa ggaggcgagg ttctaagcca ttcgctcctg 240

ctgcttcaca aaaaggaaga tggaatttgg tccactgata ttttaaagga ccagaaagaa 300

cccaaaaata agacctttct aagatgcgag gccaagaatt attctggacg tttcacctgc 360

tggtggctga cgacaatcag tactgatttg acattcagtg tcaaaagcag cagaggctct 420

tctgaccccc aaggggtgac gtgcggagct gctacactct ctgcagagag agtcagaggg 480

gacaacaagg agtatgagta ctcagtggag tgccaggagg acagtgcctg cccagctgct 540

gaggagagtc tgcccattga ggtcatggtg gatgccgttc acaagctcaa gtatgaaaac 600

tacaccagca gcttcttcat cagggacatc atcaaacctg acccacccaa gaacttgcag 660

ctgaagccat taaagaattc tcggcaggtg gaggtcagct gggagtaccc tgacacctgg 720

agtactccac attcctactt ctccctgaca ttctgcgttc aggtccaggg caagagcaag 780

agagaaaaga aagatagagt cttcacggac aagacctcag ccacggtcat ctgccgcaaa 840

aatgccagca ttagcgtgcg ggcccaggac cgctactata gctcatcttg gagcgaatgg 900

gcatctgtgc cctgcagt 918

<210> 8
<211> 591
<212> DNA
<213> Artificial Sequence

<220>
<223> IL12 P35

<400> 8
agaaacctcc ccgtggccac tccagaccca ggaatgttcc catgccttca ccactcccaa 60

aacctgctga gggccgtcag caacatgctc cagaaggcca gacaaactct agaattttac 120

ccttgcactt ctgaagagat tgatcatgaa gatatcacaa aagataaaac cagcacagtg 180

gaggcctgtt taccattgga attaaccaag aatgagagtt gcctaaattc cagagagacc 240

tctttcataa ctaatgggag ttgcctggcc tccagaaaga cctcttttat gatggccctg 300

tgccttagta gtatttatga agacttgaag atgtaccagg tggagttcaa gaccatgaat 360

gcaaagcttc tgatggatcc taagaggcag atctttctag atcaaaacat gctggcagtt 420

attgatgagc tgatgcaggc cctgaatttc aacagtgaga ctgtgccaca aaaatcctcc 480

cttgaagaac cggattttta taaaactaaa atcaagctct gcatacttct tcatgctttc 540

agaattcggg cagtgactat tgatagagtg atgagctatc tgaatgcttc c 591

<210> 9
<211> 63
<212> DNA
<213> Artificial Sequence

<220>

<223>  CD8 alpha signal peptide

<400>  9
atggccttac cagtgaccgc cttgctcctg ccgctggcct tgctgctcca cgccgccagg      60

ccg                                                                    63


<210>  10
<211>  135
<212>  DNA
<213>  Artificial Sequence

<220>
<223>  CD8 hinge region

<400>  10
accacgacgc cagcgccgcg accaccaaca ccggcgccca ccatcgcgtc gcagcccctg      60

tccctgcgcc cagaggcgtg ccggccagcg gcggggggcg cagtgcacac gagggggctg     120

gacttcgcct gtgat                                                      135


<210>  11
<211>  81
<212>  DNA
<213>  Artificial Sequence

<220>
<223>  CD28 transmembrane region

<400>  11
ttttgggtgc tggtggtggt tggtggagtc ctggcttgct atagcttgct agtaacagtg      60

gcctttatta ttttctgggt g                                                81


<210>  12
<211>  123
<212>  DNA
<213>  Artificial Sequence

<220>
<223>  CD28 intracellular region

<400>  12
aggagtaaga ggagcaggct cctgcacagt gactacatga acatgactcc ccgccgcccc      60

gggccaaccc gcaagcatta ccagccctat gccccaccac gcgacttcgc agcctatcgc     120

tcc                                                                   123


<210>  13
<211>  339
<212>  DNA
<213>  Artificial Sequence

<220>
<223>  CD3 xi domain

<400>  13

agagtgaagt tcagcaggag cgcagacgcc cccgcgtacc agcagggcca gaaccagctc 60

tataacgagc tcaatctagg acgaagagag gagtacgatg ttttggacaa gagacgtggc 120

cgggaccctg agatgggggg aaagccgcag agaaggaaga accctcagga aggcctgtac 180

aatgaactgc agaaagataa gatggcggag gcctacagtg agattgggat gaaaggcgag 240

cgccggaggg gcaaggggca cgatggcctt taccagggtc tcagtacagc caccaaggac 300

acctacgacg cccttcacat gcaggccctg ccccctcgc 339

<210> 14
<211> 20
<212> DNA
<213> Artificial Sequence

<220>
<223> primer upstream to signal sequence

<400> 14
tctcacgcgt caagtggagc 20

<210> 15
<211> 35
<212> DNA
<213> Artificial Sequence

<220>
<223> primer downstream to signal sequence

<400> 15
tctgcaccag ctgcacctcg aggtcctctt cagag 35

<210> 16
<211> 29
<212> DNA
<213> Artificial Sequence

<220>
<223> primer upstream to synNotch-GAL4VP6

<400> 16
atcctggact acagcttcac aggtggcgc 29

<210> 17
<211> 25
<212> DNA
<213> Artificial Sequence

<220>
<223> primer downstream to synNotch-GAL4VP6

<400> 17
agagccggca gcaggccgcg ggaag 25

<210> 18
<211> 642

<212>  DNA
<213>  Artificial Sequence

<220>
<223>  PGK promoter

<400>  18
gatccttgac ttgcggccgc aactcccacc tgcaacatgc gtgactgact gaggccgcga      60

ctctagagtc gacctgcatc taggcgccgg aattagatct ctcgaggtta acgaattcta     120

ccgggtaggg gaggcgcttt tcccaaggca gtctggagca tgcgctttag cagccccgct     180

gggcacttgg cgctacacaa gtggcctctg gcctcgcaca cattccacat ccaccggtag     240

gcgccaaccg gctccgttct ttggtggccc cttcgcgcca ccttctactc ctcccctagt     300

caggaagttc ccccccgccc cgcagctcgc gtcgtgcagg acgtgacaaa tggaagtagc     360

acgtctcact agtctcgtgc agatggacag caccgctgag caatggaagc gggtaggcct     420

ttggggcagc ggccaatagc agctttgctc cttcgctttc tgggctcaga ggctgggaag     480

gggtgggtcc gggggcgggc tcaggggcgg gctcaggggc ggggcgggcg cccgaaggtc     540

ctccggaggc ccggcattct gcacgcttca aaagcgcacg tctgccgcgc tgttctcctc     600

ttcctcatct ccgggccttt cgacctgcag cccaagctta cc                        642


<210>  19
<211>  30
<212>  DNA
<213>  Artificial Sequence

<220>
<223>  myc tag

<400>  19
gagcaaaaac ttatctctga agaggacctc                                       30


<210>  20
<211>  711
<212>  DNA
<213>  Artificial Sequence

<220>
<223>  mcherry

<400>  20
atggtgagca agggcgagga ggataacatg gccatcatca aggagttcat gcgcttcaag      60

gtgcacatgg agggctccgt gaacggccac gagttcgaga tcgagggcga gggcgagggc     120

cgcccctacg agggcaccca gaccgccaag ctgaaggtga ccaagggtgg ccccctgccc     180

ttcgcctggg acatcctgtc ccctcagttc atgtacggct ccaaggccta cgtgaagcac     240

cccgccgaca tccccgacta cttgaagctg tccttccccg agggcttcaa gtgggagcgc     300

gtgatgaact tcgaggacgg cggcgtggtg accgtgaccc aggactcctc cctgcaggac     360

ggcgagttca tctacaaggt gaagctgcgc ggcaccaact tcccctccga cggccccgta 420

atgcagaaga agaccatggg ctgggaggcc tcctccgagc ggatgtaccc cgaggacggc 480

gccctgaagg gcgagatcaa gcagaggctg aagctgaagg acggcggcca ctacgacgct 540

gaggtcaaga ccacctacaa ggccaagaag cccgtgcagc tgcccggcgc ctacaacgtc 600

aacatcaagt tggacatcac ctcccacaac gaggactaca ccatcgtgga acagtacgaa 660

cgcgccgagg gccgccactc caccggcggc atggacgagc tgtacaagta a 711

<210> 21
<211> 2340
<212> DNA
<213> Artificial Sequence

<220>
<223> antiGPC3-synNotch-GAL4VP64

<400> 21
gaggtgcagc tggtgcagag cggcgccgag gtgaagaagc ccggcgccag cgtgaaggtg 60

agctgcaagg ccagcggcta caccttcagc gactacgaga tgcactgggt gcggcaggcc 120

cccggccagg gcctggagtg gatgggcgcc atccaccccg gcagcggcga caccgcctac 180

aaccagcggt tcaagggccg ggtgaccatc accgccgaca agagcaccag caccgcctac 240

atggagctga gcagcctgcg gagcgaggac accgccgtgt actactgcgc ccggttctac 300

agctacgcct actggggcca gggcaccctg gtgaccgtga gcgccggtgg aggcggttca 360

ggcggaggtg gttctggcgg tggcggatcg gacatcgtga tgacccagac ccccctgagc 420

ctgcccgtga cccccggcga gcccgccagc atcagctgcc ggagcagcca gagcctggtg 480

cacagcaacg gcaacaccta cctgcagtgg tacctgcaga agcccggcca gagcccccag 540

ctgctgatct acaaggtgag caaccggttc agcggcgtgc ccgaccggtt cagcggcagc 600

ggcagcggca ccgacttcac cctgaagatc agccgggtgg aggccgagga cgtgggcgtg 660

tactactgca gccagagcat ctacgtgccc tacaccttcg gccagggcac caagctggag 720

atcaaacgta tcctggacta cagcttcaca ggtggcgctg ggcgcgacat tcccccaccg 780

cagattgagg aggcctgtga gctgcctgag tgccaggtgg atgcaggcaa taaggtctgc 840

aacctgcagt gtaataatca cgcatgtggc tgggatggtg gcgactgctc cctcaacttc 900

aatgacccct ggaagaactg cacgcagtct ctacagtgct ggaagtattt tagcgacggc 960

cactgtgaca gccagtgcaa ctcggccggc tgcctctttg atggcttcga ctgccagctc 1020

accgagggac agtgcaaccc cctgtatgac cagtactgca aggaccactt cagtgatggc 1080

cactgcgacc agggctgtaa cagtgccgaa tgtgagtggg atggcctaga ctgtgctgag 1140

catgtacccg agcggctggc agccggcacc ctggtgctgg tggtgctgct tccacccgac 1200

cagctacgga acaactcctt ccactttctg cgggagctca gccacgtgct gcacaccaac 1260

gtggtcttca agcgtgatgc gcaaggccag cagatgatct tcccgtacta tggccacgag 1320

gaagagctgc gcaagcaccc aatcaagcgc tctacagtgg gttgggccac ctcttcactg 1380

cttcctggta ccagtggtgg gcgccagcgc agggagctgg accccatgga catccgtggc 1440

tccattgtct acctggagat cgacaaccgg caatgtgtgc agtcatcctc gcagtgcttc 1500

cagagtgcca ccgatgtggc tgccttccta ggtgctcttg cgtcacttgg cagcctcaat 1560

attccttaca agattgaggc cgtgaagagt gagccggtgg agcctccgct gccctcgcag 1620

ctgcacctca tgtacgtggc agcggccgcc ttcgtgctcc tgttctttgt gggctgtggg 1680

gtgctgctgt cccgcaagcg ccggcggatg aagctgctga gcagcatcga gcaggcctgt 1740

gacatctgcc ggctgaagaa actgaagtgc agcaaagaaa agcccaagtg cgccaagtgc 1800

ctgaagaaca actgggagtg ccggtacagc cccaagacca agagaagccc cctgaccaga 1860

gcccacctga ccgaggtgga aagccggctg gaaagactgg aacagctgtt tctgctgatc 1920

ttcccacgcg aggacctgga catgatcctg aagatggaca gcctgcagga catcaaggcc 1980

ctgctgaccg gcctgttcgt gcaggacaac gtgaacaagg acgccgtgac cgacagactg 2040

gccagcgtgg aaaccgacat gcccctgacc ctgcggcagc acagaatcag cgccaccagc 2100

agcagcgagg aaagcagcaa caagggccag cggcagctga cagtgtctgc tgctgcaggc 2160

ggaagcggag gctctggcgg atctgatgcc ctggacgact tcgacctgga tatgctgggc 2220

agcgacgccc tggatgattt tgatctggac atgctgggat ctgacgctct ggacgatttc 2280

gatctcgaca tgttgggatc agatgcactg gatgactttg acctggacat gctcggatca 2340

<210> 22
<211> 3251
<212> DNA
<213> Artificial Sequence

<220>
<223> pHR-GAL4UAS-IL12-PGK-mcherry

<400> 22
tcggagcact gtcctccgaa cgtcggagca ctgtcctccg aacgtcggag cactgtcctc 60

cgaacgtcgg agcactgtcc tccgaacgga gcatgtcctc cgaacgtcgg agcactgtcc 120

tccgaacgac tagttaggcg tgtacggtgg gaggcctata taagcagagc tcgtttagtg 180

aaccgtcaga tcgcctggag acgccatcca cgctgttttg acctccatag aagacaccgg 240

gaccgatcca gcctctcgac attcgttgga tcatgtgtca ccagcagttg gtcatctctt 300

ggttttccct ggtttttctg gcatctcccc tcgtggccat atgggaactg aagaaagatg 360

tttatgtcgt agaattggat tggtatccgg atgcccctgg agaaatggtg gtcctcacct 420

gtgacacccc tgaagaagat ggtatcacct ggaccttgga ccagagcagt gaggtcttag 480

gctctggcaa aaccctgacc atccaagtca aagagtttgg agatgctggc cagtacacct 540
gtcacaaagg aggcgaggtt ctaagccatt cgctcctgct gcttcacaaa aaggaagatg 600
gaatttggtc cactgatatt ttaaaggacc agaaagaacc caaaaataag acctttctaa 660
gatgcgaggc caagaattat tctggacgtt tcacctgctg gtggctgacg acaatcagta 720
ctgatttgac attcagtgtc aaaagcagca gaggctcttc tgacccccaa ggggtgacgt 780
gcggagctgc tacactctct gcagagagag tcagagggga caacaaggag tatgagtact 840
cagtggagtg ccaggaggac agtgcctgcc cagctgctga ggagagtctg cccattgagg 900
tcatggtgga tgccgttcac aagctcaagt atgaaaacta caccagcagc ttcttcatca 960
gggacatcat caaacctgac ccacccaaga acttgcagct gaagccatta aagaattctc 1020
ggcaggtgga ggtcagctgg gagtaccctg acacctggag tactccacat tcctacttct 1080
ccctgacatt ctgcgttcag gtccagggca agagcaagag agaaaagaaa gatagagtct 1140
tcacggacaa gacctcagcc acggtcatct gccgcaaaaa tgccagcatt agcgtgcggg 1200
cccaggaccg ctactatagc tcatcttgga gcgaatgggc atctgtgccc tgcagtggtg 1260
gcggtggctc gggcggtggt gggtcgggtg gcggcggatc tagaaacctc cccgtggcca 1320
ctccagaccc aggaatgttc ccatgccttc accactccca aaacctgctg agggccgtca 1380
gcaacatgct ccagaaggcc agacaaactc tagaatttta cccttgcact tctgaagaga 1440
ttgatcatga agatatcaca aaagataaaa ccagcacagt ggaggcctgt ttaccattgg 1500
aattaaccaa gaatgagagt tgcctaaatt ccagagagac ctctttcata actaatggga 1560
gttgcctggc ctccagaaag acctctttta tgatggccct gtgccttagt agtatttatg 1620
aagacttgaa gatgtaccag gtggagttca agaccatgaa tgcaaagctt ctgatggatc 1680
ctaagaggca gatctttcta gatcaaaaca tgctggcagt tattgatgag ctgatgcagg 1740
ccctgaattt caacagtgag actgtgccac aaaaatcctc ccttgaagaa ccggattttt 1800
ataaaactaa aatcaagctc tgcatacttc ttcatgcttt cagaattcgg gcagtgacta 1860
ttgatagagt gatgagctat ctgaatgctt cctaaacgcg tgatccttga cttgcggccg 1920
caactcccac ctgcaacatg cgtgactgac tgaggccgcg actctagagt cgacctgcat 1980
ctaggcgccg gaattagatc tctcgaggtt aacgaattct accgggtagg ggaggcgctt 2040
ttcccaaggc agtctggagc atgcgcttta gcagccccgc tgggcacttg gcgctacaca 2100
agtggcctct ggcctcgcac acattccaca tccaccggta ggcgccaacc ggctccgttc 2160
tttggtggcc ccttcgcgcc accttctact cctcccctag tcaggaagtt cccccccgcc 2220
ccgcagctcg cgtcgtgcag gacgtgacaa atggaagtag cacgtctcac tagtctcgtg 2280
cagatggaca gcaccgctga gcaatggaag cgggtaggcc tttggggcag cggccaatag 2340
cagctttgct ccttcgcttt ctgggctcag aggctgggaa ggggtgggtc cgggggcggg 2400

ctcaggggcg ggctcagggg cggggcgggc gcccgaaggt cctccggagg cccggcattc 2460

tgcacgcttc aaaagcgcac gtctgccgcg ctgttctcct cttcctcatc tccgggcctt 2520

tcgacctgca gcccaagctt accatggtga gcaagggcga ggaggataac atggccatca 2580

tcaaggagtt catgcgcttc aaggtgcaca tggagggctc cgtgaacggc cacgagttcg 2640

agatcgaggg cgagggcgag ggccgcccct acgagggcac ccagaccgcc aagctgaagg 2700

tgaccaaggg tggccccctg cccttcgcct gggacatcct gtcccctcag ttcatgtacg 2760

gctccaaggc ctacgtgaag caccccgccg acatccccga ctacttgaag ctgtccttcc 2820

ccgagggctt caagtgggag cgcgtgatga acttcgagga cggcggcgtg gtgaccgtga 2880

cccaggactc ctccctgcag gacggcgagt tcatctacaa ggtgaagctg cgcggcacca 2940

acttcccctc cgacggcccc gtaatgcaga agaagaccat gggctgggag gcctcctccg 3000

agcggatgta ccccgaggac ggcgccctga agggcgagat caagcagagg ctgaagctga 3060

aggacggcgg ccactacgac gctgaggtca agaccaccta caaggccaag aagcccgtgc 3120

agctgcccgg cgcctacaac gtcaacatca agttggacat cacctcccac aacgaggact 3180

acaccatcgt ggaacagtac gaacgcgccg agggccgcca ctccaccggc ggcatggacg 3240

agctgtacaa g 3251

<210> 23
<211> 469
<212> PRT
<213> Artificial Sequence

<220>
<223> GPC-28Z

<400> 23

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys

                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Arg Val Lys Phe Ser Arg Ser Ala Asp Ala Pro Ala
        355                 360                 365

Tyr Gln Gln Gly Gln Asn Gln Leu Tyr Asn Glu Leu Asn Leu Gly Arg
    370                 375                 380

Arg Glu Glu Tyr Asp Val Leu Asp Lys Arg Arg Gly Arg Asp Pro Glu
385                 390                 395                 400

Met Gly Gly Lys Pro Gln Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465

<210>  24
<211>  466
<212>  PRT
<213>  Artificial Sequence

<220>
<223>  GPC-BBZ

<400>  24

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr

65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Ile Tyr Ile Trp Ala Pro Leu Ala Gly Thr Cys Gly Val Leu Leu Leu
    290                 295                 300

Ser Leu Val Ile Thr Leu Tyr Cys Lys Arg Gly Arg Lys Lys Leu Leu
305                 310                 315                 320

Tyr Ile Phe Lys Gln Pro Phe Met Arg Pro Val Gln Thr Thr Gln Glu
                325                 330                 335

Glu Asp Gly Cys Ser Cys Arg Phe Pro Glu Glu Glu Glu Gly Gly Cys
            340                 345                 350

Glu Leu Arg Val Lys Phe Ser Arg Ser Ala Asp Ala Pro Ala Tyr Lys
        355                 360                 365

Gln Gly Gln Asn Gln Leu Tyr Asn Glu Leu Asn Leu Gly Arg Arg Glu
    370                 375                 380

Glu Tyr Asp Val Leu Asp Lys Arg Arg Gly Arg Asp Pro Glu Met Gly
385                 390                 395                 400

Gly Lys Pro Arg Arg Lys Asn Pro Gln Glu Gly Leu Tyr Asn Glu Leu
                405                 410                 415

Gln Lys Asp Lys Met Ala Glu Ala Tyr Ser Glu Ile Gly Met Lys Gly
            420                 425                 430

Glu Arg Arg Arg Gly Lys Gly His Asp Gly Leu Tyr Gln Gly Leu Ser
        435                 440                 445

Thr Ala Thr Lys Asp Thr Tyr Asp Ala Leu His Met Gln Ala Leu Pro
    450                 455                 460

Pro Arg
465

<210>  25
<211>  511
<212>  PRT
<213>  Artificial Sequence

<220>
<223>  GPC-28BBZ

<400>  25

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe

50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Lys Arg Gly Arg Lys Lys Leu Leu Tyr Ile Phe Lys
        355                 360                 365

Gln Pro Phe Met Arg Pro Val Gln Thr Thr Gln Glu Glu Asp Gly Cys
    370                 375                 380

Ser Cys Arg Phe Pro Glu Glu Glu Glu Gly Gly Cys Glu Leu Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Gln
        435                 440                 445

Arg Arg Lys Asn Pro Gln Glu Gly Leu Tyr Asn Glu Leu Gln Lys Asp
    450                 455                 460

Lys Met Ala Glu Ala Tyr Ser Glu Ile Gly Met Lys Gly Glu Arg Arg
465                 470                 475                 480

Arg Gly Lys Gly His Asp Gly Leu Tyr Gln Gly Leu Ser Thr Ala Thr
                485                 490                 495

Lys Asp Thr Tyr Asp Ala Leu His Met Gln Ala Leu Pro Pro Arg
            500                 505                 510

<210>  26
<211>  356
<212>  PRT
<213>  Artificial Sequence

<220>
<223>  GPC3-z

<400>  26

Glu Val Gln Leu Val Gln Ser Gly Ala Glu Val Lys Lys Pro Gly Ala

1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Arg Val Lys Phe Ser Arg Ser Ala Asp Ala Pro Ala Tyr
                245                 250                 255

Gln Gln Gly Gln Asn Gln Leu Tyr Asn Glu Leu Asn Leu Gly Arg Arg
            260                 265                 270

Glu Glu Tyr Asp Val Leu Asp Lys Arg Arg Gly Arg Asp Pro Glu Met
        275                 280                 285

Gly Gly Lys Pro Gln Arg Arg Lys Asn Pro Gln Glu Gly Leu Tyr Asn
    290                 295                 300

Glu Leu Gln Lys Asp Lys Met Ala Glu Ala Tyr Ser Glu Ile Gly Met
305                 310                 315                 320

Lys Gly Glu Arg Arg Arg Gly Lys Gly His Asp Gly Leu Tyr Gln Gly
                325                 330                 335

Leu Ser Thr Ala Thr Lys Asp Thr Tyr Asp Ala Leu His Met Gln Ala
            340                 345                 350

Leu Pro Pro Arg
        355

<210> 27
<211> 540
<212> PRT
<213> Artificial Sequence

<220>
<223> amino acid sequence of human IL12

<400> 27

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp

100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
            340                 345                 350

Pro Gly Met Phe Pro Cys Leu His His Ser Gln Asn Leu Leu Arg Ala
        355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
            420                 425                 430

Ala Ser Arg Lys Thr Ser Phe Met Met Ala Leu Cys Leu Ser Ser Ile
        435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser
    530                 535                 540

<210>  28
<211>  22
<212>  DNA
<213>  Artificial Sequence

<220>
<223>  primer upstream to GAL4UAS

<400>  28
agatcgcgat gggaaaaat tc                                                22

<210>  29
<211>  37
<212>  DNA

<213>  Artificial Sequence

<220>
<223>  primer downstream to GAL4UAS

<400>  29
tgctgaactt cactctcatg atccaacgaa tgtcgag                                37

## Description

The present application claims the priority of CN201810053219.6 filed on January 19, 2018, the entire contents of which are incorporated herein by reference.

### Technical field

The invention belongs to the field of immunotherapy, and in particular relates to a pair of plasmid vectors and an immune cell that target antigen-dependently regulates the expression of IL12.

### Background

IL12 is a kind of immune cell growth stimulating factor with multiple biological activities and a heterodimeric cytokine that can promote the proliferation of T helper cells 1 (Th1); induce NK cells and T cells to produce interferon gamma; improve the cytotoxicity of NK cells; and promote the formation of cytotoxic T cells. At present, IL12 is directly loaded to enhance the anti-tumor efficacy of a chimeric antigen receptor (CAR), however, IL12 will be systemically secreted with CAR-T by such manner, and the expression of IL12 will be out of controll with the amplification of T cells, thereby causing serious toxic and side effects to the body.

U.S. Patent No. 9,834,608 B2 (the patent is incorporated herein by reference in its entirety) disclosed a binding-triggered transcription switch polypeptide and an encoding nucleic acid and host cell thereof. The polypeptide is a chimeric Notch receptor polypeptide, including an extracellular domain, a Notch regulatory region and an intracellular domain, wherein the extracellular domain contains a first member of a specific binding pair, which specifically binds to a second member of the specific binding pair, the Notch regulatory region contains Lin 12-Notch repeat unit, S2 proteolytic cleavage site and the transmembrane domain comprisng S3 proteolytic cleavage site, and the first member of the specific binding pair binds to the second member to induce cleavage at S2 and S3 proteolytic cleavage sites, thereby releasing the intracellular domain. The first member is selected from the group consisting of an antibody, an antibody-based recognition scaffold, a non-antibody-based recognition scaffold, an antigen, a ligand of a receptor, a receptor, a target for a non-antibody-based recognition scaffold, an extracellular matrix component, and an adhesion molecule. The intracellular domain contains a transcriptional activator, and the release of the intracellular domain causes the expression of an endogenous gene in cells induced by the transcriptional activator. Alternatively, the intracellular domain contains a transcription repressor, and the release of the intracellular domain inhibit the expression of an endogenous gene in cells by the transcription repressor.

However, the necessity for controlling the expression and local secretion of IL-12 in immune cells and caused beneficial effects are not mentioned in this US patent.

### Summary of the invention

In the prior art, no immune cells can control expression and local secretion of IL-12. For resolving such technical problem, a pair of plasmid vectors and an immune cell that target antigen-dependently regulates the expression of IL12 through synNotch technology are provided in the present invention. In particular, in the present invention, a synthetic Notch (synNotch) receptor was used as a core in NK92 cells to construct two plasmid vectors for achieving local secretion of IL12. In one of the plasmid vectors, antiGPC3 scfv (SEQ ID NO: 2) is used as an extracellular segment to recognize the target antigen GPC3; the notch core (SEQ ID NO: 3) includes a transmembrane region and contains two enzyme cleavage sites; and GAL4VP64 (SEQ ID NO: 4) transcription factor is used as an intracellular segment. When the target antigen GPC3 is recognized, the cleavage site on the notch core will be recognized and hydrolyzed by the corresponding enzyme and the intracellular transcription factor GAL4VP64 will be released. Another plasmid vector carries the DNA recognition binding region GAL4UAS (SEQ ID NO: 5) of the GAL4 transcription factor and the corresponding minimal CMV promoter (SEQ ID NO: 6) that initiates the target gene IL12. When GAL4VP64 is hydrolyzed and cleaved, it will bind to the binding region GAL4UAS, thereby initiating the transcription of subsequent gene, expressing and secreting IL12 out of the cell.

The generation of toxicity can be reduced by the present invention, since the secretion and expression of IL12 can be induced only when exposed to a specific target antigen, thereby locally releasing IL12 near the tumor and enhancing the effects of CAR-T cells. Moreover, the used NK92 cell line will not substantially expand *in vivo*, thereby avoiding serious toxic and side effects caused by the uncontrolled expression of IL12 induced by T cell expansion *in vivo.* In addition, compared with NK cells, NK92 can be mass-produced, which is more suitable for industrial applications.

In one aspect, the present invention relates to a pair of plasmid vectors including a first vector and a second vector, wherein the first vector comprises a Notch core and further comprises encoding nucleic acid molecules for other extracellular segments and intracellular segments, and preferably, the intracellular segment comprises transcriptional activator; and the second vector comprises a nucleic acid molecule that specifically recognizes and binds to the intracellular segment in the first vector and a nucleic acid molecule encoding IL12.

In a preferred embodiment, the Notch core of the first vector has the nucleic acid sequence as shown in SEQ ID NO: 3.

According to the present invention, the intracellular segment of the first vector comprises a transcriptional activator, such as a tetracycline-controlled transcriptional activator (tTa), GAL4VP64 and ZFHD1-VP64, and the second vector comprises a DNA binding region corresponding to the transcriptional activator, for example, Tet, UAS and ZFRE. Preferably, the intracellular segment of the first vector comprises GAL4VP64 and the second vector comprises GAL4UAS. The intracellular segment of the first vector is a DNA-binding polypeptide, such as Zip(-)Gal4 DNA-binding polypeptide, or NLS VP64 Zip (+).

The extracellular segment in the first vector of the present invention is not particularly limited, as long as it can specifically bind to and pair with a target, for example, it may comprise an antigen or an antibody or fragment thereof of the antigen, a receptor or a ligand of the receptor, a non-antibody-based recognition scaffold or a target thereof, an adhesion molecule or an extracellular matrix thereof, Fc or a receptor thereof, a receptor or a co-receptor thereof, etc.

The antibodies of the present invention specifically bind to an antigen, including nanobodies, single chain antibodies, bivalent antibodies, trivalent antibodies or mini antibodies. An antibody fragment refers to a part of an intact antibody, such as the antigen binding region or variable region of the intact antibody. The antibody fragment includes Fab, Fab', F(ab')2 and Fv fragment. Fv is the smallest antibody fragment that contains a complete antigen recognition and binding site. Single chain Fv (scFv) refers to the VH and VL domains of an antibody, these domains take the form of an single chain polypeptide. In some embodiments, the Fv polypeptide also comprises a linker between VH and VL. The extracellular segment preferably comprises an antibody, and more preferably comprises scFv.

The antibody of the present invention or a fragment thereof is specifically directed to an epitope on an antigen, especially an antigen associated with a cancer cell, including breast cancer, B-cell lymphoma, prostate cancer, Hodgkin lymphoma, ovarian cancer, and lung cancer (e.g., small cell lung cancer), mesothelioma, melanoma, acute and chronic lymphocytic leukemia, neuroblastoma, glioma, glioblastoma, medulloblastoma, colon cancer, gastric cancer, liver cancer, etc.

According to the present invention, the tumor antigens include one or more from prostate specific membrane antigen (PSMA), carcinoembryonic antigen (CEA), IL13Ralpha, HER-2, CD19, NY-ESO-1, HIV-1 Gag, Lewis Y, MART-1 , Gp100, tyrosinase, WT-I, hTERT, mesothelin, EGFR, EGFRvIII, GPC3, EphA2, HER3, EpCAM, MUC1, MUC16, CLDN18.2, folate receptor, CLDN6, CD30, CD138, ASGPR1 CDH16, GD2, 5T4, 8H9, αvβ6 integrin, B cell maturation antigen (BCMA), B7-H3, B7-H6, CAIX, CA9, CD20, CD22, kappa light chain, CD33, CD38, CD44 , CD44v6, CD44v7/8, CD70, CD123, CD171, CSPG4, EGP2, EGP40, ERBB3, ERBB4, ErbB3/4, FAP, FAR, FBP, embryonic AchR, GD2, GD3, HLA-AI MAGE A1, MAGE3, HLA -A2, IL11Ra, KDR, Lambda, MCSP, NCAM, NKG2D ligand, PRAME, PSCA, PSC1, ROR1, Sp17, SURVIVIN, TAG72, TEM1, TEM8, VEGRR2, HMW-MAA, VEGF receptor, and/or fibronectin, cytotactin or carcinoembryogenic variants of tumor necrosis region.

According to the present invention, the extracellular segment includes, but is not limited to, for example, anti-CD19 scFv, anti-mesothelin scFv, anti-GFP nanobody, anti-GPC3 scFv, etc., preferably, anti-GPC3 scFv (i.e., anti-GPC3 scFv).

According to the present invention, the Notch core includes a Notch transmembrane region and one or more cleavage sites.

According to the present invention, one or more epidermal growth factor (EGF) repeating units can optionally be added to the extracellular end of the Notch core. The EGF repeat unit can reduce the non-specific activation of the basic background.

According to the present invention, the first vector and the second vector optionally comprise elements, such as a signal sequence (for example, SEQ ID NO: 1), a linker, a promoter, a tag, and a reporter, wherein the tag includes one or more of blood coagulation HA, C-myc and Flag, and the like. The reporter includes one or more of green fluorescent protein (GFP), blue fluorescent protein (BFP) and mcherry, and the like.

According to the present invention, the first vector sequentially comprises the PGK promoter - signal sequence - myc tag - anti-GPC3 scFv - notch core - GAL4 - linker - VP64, and the second vector sequentially comprises the GAL4UAS - CMV minimal promoter - IL12 - PGK promoter - mcherry.

According to the present invention, the open reading frame of the first vector comprises a nucleotide sequence as shown in SEQ ID NO: 21, and the open reading frame of the second vector comprises a nucleotide as shown in SEQ ID NO: 22. The amino acid sequences encoded by the above nucleotide sequences are the elements of the vectors of the present invention and/or the polypeptides encoded by the vectors.

According to the invention, the function of a polypeptide variant generally won't be substantially affected by the substitution, deletion and/or addition of one or more amino acids, especially when the conservative regions of the polypeptide are known. Conservative substitutions (for example, one basic amino acid for another basic amino acid and one acidic amino acid for another acidic amino acid) are also readily conceived by a skilled person in the art. Therefore, in addition to the elements of the vector of the invention and / or the amino acid sequence of the polypeptide encoded by the vector, functional variants having at least 50%, preferably 70%, 75%, 80% , 85% or 90%, more preferably 95%, 96%, 97%, 98%, 99% identity with the elements of the vector of the invention and / or the amino acid sequence of the polypeptide encoded by the vector will fall within the protection scope. More over, nucleotide codons encoding the polypeptides may be degenerate, and therefore functional variants having at least 50%, preferably 70%, 75%, 80%, 85%, or 90% %, more preferably 95%, 96%, 97%, 98% or 99% identity with the elements of the vector of the invention and / or the nucleotide sequence of the vector also fall within the protection scope. Even more preferably, the anti-GPC3 scFv has at least 90% identity with the nucleotide sequence as shown in SEQ ID NO: 2.

In another aspect, the present invention also provides immune cells modified with the pair of plasmid vectors of the present invention. The immune cells are T cells or NK cells. Preferably, the immune cells are NK cells. The cells are transfected with any of the above pairs of the plasmid vectors. The NK cells are natural NK cells or immortalized NK cells, preferably, immortalized NK cells, more preferably, NK92 cell.

In another aspect, the present invention provides uses of any of the above pairs of the plasmid vectors or any of the above immune cells in the preparation of a medicament for local secretion of IL12 or regulating expression of IL12.

In yet another aspect, the present invention provides uses of any of the above pairs of plasmid vectors or any of the above immune cells in the preparation of a medicament for enhancing killing effects of CAR-T cells.

In a preferred embodiment, the tumor antigen specifically recognized by the extracellular segment of the first vector of the immune cell is the same as the tumor antigen recognized by the CAR-T cell.

In a preferred embodiment, both of the tumor antigen specifically recognized by the extracellular segment of the first vector of the immune cell and the tumor antigen recognized by the CAR-T cell are expressed on the same tumor.

In a preferred embodiment, both of the tumor antigen specifically recognized by the extracellular segment of the first vector of the immune cell and the tumor antigen recognized by the CAR-T cells are GPC3.

In a preferred embodiment, both of the tumor antigen specifically recognized by the extracellular segment of the first vector of the immune cell and the tumor antigen recognized by the CAR-T cells are expressed in liver cancer.

In a preferred embodiment, the first vector of the immune cells comprises the sequence as shown in SEQ ID NO: 21, and the second vector comprises the sequence as shown in SEQ ID NO: 22;

In a preferred embodiment, the CAR of the CAR-T cell has the sequence as shown in SEQ ID NO: 23, 24 or 25.

In a preferred embodiment, the ratio of the immune cells to CAR-T cells is 1: 1 ∼ 4: 1.

In a preferred embodiment, the immune cells can be administrated in multiple times within one administration cycle.

In yet another aspect, the present invention provides a composition of the immune cells and CAR-T cells as described.

In a preferred embodiment, the tumor antigen specifically recognized by the extracellular segment of the first vector of the immune cell is the same as the tumor antigen recognized by the CAR-T cell.

In a preferred embodiment, both of the tumor antigen specifically recognized by the extracellular segment of the first vector of the immune cell and the tumor antigen recognized by the CAR-T cell are expressed on the same tumor.

In a preferred embodiment, both of the tumor antigen specifically recognized by the extracellular segment of the first vector of the immune cell and the tumor antigen recognized by the CAR-T cell are GPC3.

In a preferred embodiment, both of the tumor antigen specifically recognized by the extracellular segment of the first vector of the immune cell and the tumor antigen recognized by the CAR-T cell are expressed in liver cancer.

In a preferred embodiment, the first vector of the immune cell comprises the sequence as shown in SEQ ID NO: 21, the second vector comprises the sequence as shown in SEQ ID NO: 22, and the CAR of the CAR-T cell comprises the sequence as shown in SEQ ID NO: 23, 24 or 25.

In a preferred embodiment, the ratio of the immune cells to the CAR-T cells is 1: 1 ∼ 4: 1.

In a final aspect, the present invention also relates to a combination of kits, including kit A of the immune cell and kit B containing the CAR-T cell as described above.

In a preferred embodiment, the tumor antigen specifically recognized by the extracellular domain of the first vector of the immune cell is the same as the tumor antigen recognized by the CAR-T cell.

In a preferred embodiment, both of the tumor antigen specifically recognized by the extracellular domain of the first vector of the immune cell and the tumor antigen recognized by the CAR-T cell are expressed on the same tumor.

In a preferred embodiment, both of the tumor antigen specifically recognized by the extracellular domain of the first vector of the immune cell and the tumor antigen recognized by the CAR-T cell are GPC3.

In a preferred embodiment, both of the tumor antigen specifically recognized by the extracellular domain of the first vector of the immune cell and the tumor antigen recognized by the CAR-T cell are expressed in liver cancer.

In a preferred embodiment, the first vector of the immune cell comprises the sequence as shown in SEQ ID NO: 21, the second vector comprises the sequence as shown in SEQ ID NO: 22, and the CAR of the CAR-T cell comprises the sequence as shown in SEQ ID NO: 23, 24 or 25.

In the present invention, it is demonstrated through experiments that uninfected NK92 cells secrete a low level of IL12, while the secretion of IL12 from pHRLSIN-antiGPC3-synNotch-GAL4VP64 and pHRLSIN-GAL4UAS -IL12-PGK-mcherry double-infected NK92 cells significantly increases, when co-incubated with cell lines highly expressing GPC3, respectively.

Therefore, the single-chain antibodies against GPC3 can regulate the expression of cytokine IL12 in NK92 cells and can locally release IL12.

### Description of figures

In order to clearly describe the technical solution of the present invention, a brief introduction will be given below with reference to the drawings. These drawings are obviously only some specific embodiments described in this application. The invention includes, but is not limited to these drawings.
Figure 1: Construction of two vectors;
Figure 2: Positive rate of infected NK92 cells;
Figure 3: Expression of GPC3 of target cells;
Figure 4A shows the expression of cytokine IL12 detected by Elisa;
Figure 4B shows the secretion of IL12 at different time points;
Figure 5A shows the tumor growth curve in mice;
Figure 5B shows the comparison results of tumor weights in mice;
Figure 5C shows the weight change of the mouse xenograft model under different administration conditions;
Figure 6 shows the results of immunohistochemistry;
Figure 7 shows the killing effects on cells *in vitro.*

### Modes for carrying out the invention

In order to further understand the present invention, the preferred solutions of the present invention will be described below in conjunction with embodiments. These descriptions are only illustrative of the features and advantages of the present invention, rather than limiting the protection scope of the present invention. If the conditions are not specified in the following examples, the experimental method is performed according to the conventional conditions, such as those described in Sambrook et al. "Molecular Cloning: Experimental Manual (1989)", or the conditions recommended by the instructions from reagent companies.

The following detailed description shows in detail the embodiments disclosed herein. It should be understood that this description is not limited to the specific embodiments disclosed herein, which may vary. A skilled person in the art will understand that there may be many changes or variations for the contents disclosed in this specification, which are covered within the disclosed scope and principles. Unless otherwise stated, each embodiment can be arbitrarily combined with any other embodiment.

Certain embodiments disclosed herein include numerical ranges, and certain aspects of the invention can be described in terms of ranges. Unless otherwise stated, it should be understood that the numerical range or description in range is just for brevity and convenience, and should not be considered as a strict limitation on the scope of the present invention. Therefore, a description in range should be considered as specifically disclosing all possible sub-ranges and all possible specific numerical points within the range, as these sub-ranges and numerical points have been explicitly written herein. For example, the description of a range from 1 to 6 should be considered to specifically disclose subranges from 1 to 3, 1 to 4, 1 to 5, 2 to 4, 2 to 6, 3 to 6, etc., and specific numerical points within these ranges, such as 1, 2, 3, 4, 5, 6. Regardless of the scope of the value, the above principles are equally applicable. When a range is descrbed, the endpoints of the range shall be included.

The term "receptor" is a type of special protein that exists in the cell membrane or intracellularly, and can combine with a special signaling molecule outside the cell to activate a series of biochemical reactions in the cell and cause the cell to produce corresponding effects to external stimuli. Biologically active substances that bind to a receptor are collectively called ligands.

The term "chimeric antigen receptor" or "CAR" refers to an engineered molecule that can be expressed by immune cells including but not limited to T cells. CAR is expressed in T cells and can redirect the T cells to induce killing effects on target cells with specificity determined by artificial receptors. The extracellular binding domain of CAR can be derived from murine, humanized or fully human monoclonal antibodies.

A chimeric antigen receptor usually comprises an extracellular antigen binding region. In some embodiments, the extracellular antigen binding region may be fully of human. In other cases, the extracellular antigen binding region can be humanized. In other cases, the extracellular antigen binding region may be of murine origin, or the chimera in the extracellular antigen binding region consists of amino acid sequences derived from at least two different animals. In some embodiments, the extracellular antigen binding region may be of non-human, or multiple antigen-binding regions may be designed. Non-limiting examples include single-chain variable fragments (scFv) derived from antibodies, fragment antigen binding regions (Fab) selected from libraries, single-domain fragments, or natural ligands conjugated to cognate receptors thereof. In some embodiments, the extracellular antigen binding region may comprise scFv, Fab or natural ligand, and any derivatives thereof. The extracellular antigen binding region may refer to a molecule other than the intact antibody, which may comprise a part of the intact antibody and may bind to the antigen to which the intact antibody binds. Examples of antibody fragments may include, but are not limited to, Fv, Fab, Fab', Fab'-SH, F(ab')2; bifunctional antibodies, linear antibodies; single-chain antibody molecules (e.g., scFv); and multispecific antibodies formed from antibody fragments.

The term "engineered" and other grammatical forms thereof may refer to one or more changes of nucleic acids, such as nucleic acids within the genome of an organism. The term "engineered" may refer to a change, addition and/or deletion of a gene. Engineered cells can also refer to cells that contain added, deleted, and/or changed genes.

The term "cell" or "engineered cell" and other grammatical forms thereof may refer to a cell derived from human or non-human animal. Engineering cells can also refer to cells that express CAR

The term "transfection" refers to the introduction of a foreign nucleic acid into an eukaryotic cell. Transfection can be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retrovirus infection, and biolistics.

As used herein, the terms "encoding nucleic acid molecule" and "encoding nucleic acid sequence" refer to the order of deoxyribonucleotides along a deoxyribonucleic acid chain, and the order of these deoxyribonucleotides determines the order of amino acids in a polypeptide (protein) chain. Therefore, the nucleic acid sequence encodes an amino acid sequence.

The term "peripheral blood lymphocytes" and other grammatical forms thereof may refer to lymphocytes circulating in blood (e.g., peripheral blood). Peripheral blood lymphocytes may refer to lymphocytes not limited in organs. The peripheral blood lymphocytes may comprise T cells, NK cells, B cells, or any combination thereof.

The term "immune cell" refers to a cell that can elicit an immune response, including but not limited to T cells, B cells, NK cells, NKT cells, DNT cells, etc., their respective precursor cells and progenies. Immune response cells can also refer to cells of the lymphoid or bone marrow lineage.

The term "immune cell" and other grammatical forms thereof may refer to immune cells of any origin. For example, immune cells may be derived from blood, such as autologous T cells, allogeneic T cells, autologous NK cells, xenogeneic NK cells, or may be derived from cell lines, such as NK cell line prepared by infection with EBV virus, NK cells and NK92 cell line induced from embryonic stem cells and iPSC.

The term "immune cell" may also be of human or non-human.

When used to refer to a nucleotide sequence, the term "sequence" and other grammatical forms thereof may include DNA or RNA, and may be single-stranded or double-stranded. The nucleic acid sequence can be mutated. The nucleic acid sequence can be of any length.

The term "effective amount" refers to an amount that provides therapeutic or preventive benefits.

The term "promoter" as used herein is defined as a DNA sequence that is recognized by the cell's synthetic mechanism or the introduced synthetic mechanism required to initiate specific transcription of the polynucleotide sequence.

The term "vector" as used herein is a composition that comprises an isolated nucleic acid and can be used to deliver the isolated nucleic acid inside a cell. Many vectors are known in the art, including but not limited to linear polynucleotides, polynucleotides related to ionic or amphiphilic compounds, plasmids, and viruses. Therefore, the term "vector" includes autonomously replicating plasmids or viruses. The term should also be interpreted to include non-plasmid and non-viral compounds facilitating the transfer of nucleic acids into cells, such as polylysine compounds, liposomes, and the like. Examples of viral vectors include, but are not limited to, adenovirus vectors, adeno-associated virus vectors, retrovirus vectors, and the like.

The term sequence "identity" as used herein determines the percent identity by comparing two best-matched sequences on a comparison window, where portions of the polynucleotide or polypeptide sequences in the comparison window may contain additions or deletions (i.e., gaps), for example, for two sequences that best match, a gap of 20% or less (e.g., 5 to 15%, or 10 to 12%) compared with a reference sequence (which does not include an addition or deletion). The percentage is usually calculated by determining the number of positions where the same nucleic acid base or amino acid residue is present in the two sequences to produce the number of correctly matched positions, and dividing the number of correctly matched positions by the total number of positions in the reference sequence (i.e., window size), and multiply the result by 100 to produce the percentage of sequence identity.

The terms "antiGPC3", "anti-GPC3" and "anti GPC3" can be used interchangeably and have the same meaning.

The terms "IL12", "Interleukin 12" and "interleukin-12" can be used interchangeably and have the same meaning. The term "IL12" is an immunomodulatory factor with multiple biological activities. For example, the term "IL12" may refer to human IL12 as defined in SEQ ID NO: 27 or active fragments thereof, or may be from other species.

In some embodiments, the elements used to construct the receptor specifically binding to GPC3 or IL12 may be naturally occurring. For example, it may be isolated or purified from a mammal; it may also be artificially prepared, such as the elements or IL12 can be recombination-produced according to conventional genetic engineering recombination technology. Preferably, recombinant elements or IL12 can be used in the present invention.

Amino acid sequences formed by substitution, deletion, or addition of one or more amino acid residues based on the aforementioned elements or IL12 polypeptide sequence are also included in the present invention. Proper replacement of amino acids is a technique well known in the art, which can be easily implemented and ensure that biological activities of the resulting molecule will not be altered. According to these techniques, a skilled person in the art will appreciate that changing a single amino acid in a non-essential region of a polypeptide will generally not substantially alter biological activities thereof.

Each of the elements or the biologically active fragments of IL12 polypeptide can be used in the present invention. The biologically active fragment herein means a polypeptide, as a part of a full-length polypeptide, that can maintain all or part of the functions of the full-length polypeptide. The biologically active fragment generally maintains at least 50% of the activity of the full-length polypeptide. In a preferred embodiment, the active fragment can maintain 60%, 70%, 80%, 90%, 95%, 99%, or 100% of the activity of the full-length polypeptide.

Modified or improved polypeptides can also be used in the present invention based on the elements or IL12 polypeptide sequence, for example, a polypeptide modified or improved for promoting half-life, effectiveness, metabolism, and/or efficacy of the polypeptide. That is, any variation forms not affecting the biological activity of the polypeptide can be used in the present invention.

The term "tumor" refers to a disease characterized by a pathological proliferation of cells or tissues, and the subsequent migration or invasion thereof to other tissues or organs. The growth of a tumor is usually uncontrolled and progressive, and does not induce or inhibit the proliferation of normal cells. Tumors can affect a variety of cells, tissues or organs, including but not limited to bladder, breast, esophagus, intestine, kidney, liver, lung, lymph nodes, nervous tissue, ovary, pancreas, prostate, skeletal muscle, skin, spinal cord, spleen, stomach, uterine organs, or tissues or corresponding cells. The tumors of the present invention may include, but are not limited to, liver cancer, stomach cancer, lung cancer, breast cancer, head and neck cancer, bladder cancer, ovarian cancer, cervical cancer, renal cancer, pancreatic cancer, cervical cancer, liposarcoma, melanoma, adrenal gland cancer, schwannoma, malignant fibrous histiocytoma, esophageal cancer. Preferably, the "tumor" includes but is not limited to: liver cancer, gastric cancer, lung cancer, breast cancer.

The extracellular antigen binding region can bind to any complementary target. The extracellular antigen binding region can be derived from antibodies with known variable region sequences. The extracellular antigen binding region can be obtained from antibody sequences obtained from available mouse hybridomas. Alternatively, the extracellular antigen-binding region can be obtained from whole external cleavage sequencing of tumor cells or primary cells, such as tumor infiltrating lymphocytes (TIL).

In some cases, the binding specificity of extracellular antigen binding regions can be determined by complementarity determining regions or CDRs, such as light chain CDRs or heavy chain CDRs. In many cases, the binding specificity can be determined by the light chain CDR and the heavy chain CDR. Compared with other reference antigens, a given binding pocket can be provided by a given combination of heavy and light chain CDRs, which can confer greater affinity and/or specificity for the antigen (e.g., GPC3). For example, CDRs specific for Glypican-3 can be expressed in the extracellular binding region of CAR, so that CAR targeting GPC3 can target immune response cells to tumor cells expressing GPC3.

In certain aspects of any embodiment disclosed herein, the extracellular antigen binding region, such as scFv, may comprise light chain CDRs specific for an antigen. The light chain CDR may be the complementarity determining region of scFv light chain of an antigen binding unit, such as CAR. The light chain CDR may comprise a continuous sequence of amino acid residues, or two or more continuous sequences of amino acid residues separated by non-complementarity determining regions (e.g., framework region). In some cases, the light chain CDR may comprises two or more light chain CDRs, which may be named as light chain CDR-1, CDR-2, etc. In some cases, the light chain CDR may comprise three light chain CDRs, which may be named as light chain CDR-1, light chain CDR-2, and light chain CDR-3, respectively. In some embodiments, a group of CDRs present on a common light chain may be collectively named as light chain CDRs.

In certain aspects of any embodiments disclosed herein, the extracellular antigen-binding region, such as scFv, may comprises heavy chain CDRs specific for an antigen. The heavy chain CDR may be a heavy chain complementarity determining region of an antigen binding unit, such as scFv. The heavy chain CDR may comprise a continuous sequence of amino acid residues, or two or more continuous sequences of amino acid residues separated by non-complementarity determining regions (e.g., framework region). In some cases, the heavy chain CDR may comprise two or more heavy chain CDRs, which may be named as heavy chain CDR-1, CDR-2, and the like. In some cases, the heavy chain CDR may comprise three heavy chain CDRs, which may be named as heavy chain CDR-1, heavy chain CDR-2, and heavy chain CDR-3, respectively. In some cases, a group of CDRs present on a common heavy chain may be collectively named as heavy chain CDRs.

The extracellular antigen-binding region can be modified in various ways through genetic engineering. In some cases, the extracellular antigen binding region can be mutated so that the extracellular antigen binding region can be selected to have a higher affinity for a target. In some cases, the affinity of the extracellular antigen-binding region to a target can be optimized for the target expressed at low levels on normal tissues. Such optimization can be performed to minimize potential toxicity. In other cases, clones of the extracellular antigen-binding region with higher affinity for the membrane-bound form of the target may be superior to the soluble form counterpart. Such modifications can be conducted since different levels of the target in soluble form can also be detected, and undesirable toxicities can be caused by such targeting.

In some cases, the extracellular antigen-binding region includes hinges or spacers. The terms hinge and spacer can be used interchangeably. The hinge may be considered as a part for providing flexibility to the extracellular antigen binding region.

The term "treatment" refers to a clinical intervention trying to change an individual or processing a disease caused by cells, which can be of preventive and performed during the clinical pathological process. Therapeutic effects include, but not limited to, preventing the occurrence or recurrence of a disease, reducing symptoms, reducing the direct or indirect pathological consequences of any disease, preventing metastasis, slowing the progress of a disease, improving or alleviating conditions, alleviating or improving prognosis, etc.

The term "constitutive expression" refers to an expression under all physiological conditions.

The term "inducible expression" refers to an expression under certain conditions, for example when T cells bind to an antigen. A skilled person will know how to perform a conventional "inducoble expression".

The term "Notch receptor" includes 3 key components: 1) ligand binding epidermal growth factor (EGF) repeat sequence, 2) notch core that controls the cleavage of the receptor during activation, and 3) intracellular domain which is released and regulates the transcription.

The term "Notch core" is the smallest scaffold that controls ligand-dependent cleavage and activation, including Lin12-Notch repeat sequence (LNR) that controls the accessibility of the S2 cleavage site to metalloproteases, heterodimerization domain (HD), and transmembrane domain (TMD) comprising γ-secretase cleavage site required for the release of Notch intracellular domain (NICD).

### Immune cells

In some embodiments, the present invention provides an immune cell expressing a pair of plasmid vectors.

In some embodiments, the immune cell is capable of locally secreting IL12 or regulating the expression of IL12.

In some embodiments, the immune cell is capable of enhancing tumor killing effects of CAR-T cells.

### Composition

The immune cells of the present invention can be used in combination with CAR-T cells. In addition to an effective amount of immune cells, the pharmaceutical composition may also comprise an effective amount of CAR-T cells.

### Combination with anti-tumor drugs

In some embodiments, the immune cells of the invention can be administered in combination with another therapeutic agent. In some embodiments, the another therapeutic agent is a chemotherapeutic agent. Chemotherapy drugs that can be used in combination with the immune cells of the present invention include, but not limited to, mitotic inhibitors (vinca alkaloids), including vincristine, vinblastine, vindesine, and novibin (TM) (vinorelbine , 5'-dehydrohydrogen sulfide); topoisomerase I inhibitors, such as camptothecin compounds, including Camptosar™ (Irinotecan HCL), Hycamtin™ (Topotecan HCL) and other compounds derived from camptothecin and analogs thereof; podophyllotoxin derivatives, such as etoposide, teniposide, and midoxazole; alkylating agents cisplatin, cyclophosphamide, nitrogen mustard, trimethylene thiophosphoramide, Carmustine, busulfan, chlorambucil, buquinazine, uracil mustard, cloprofen, and dacarbazine; antimetabolites, including cytarabine, fluorouracil, methotrexate, mercaptopurine, azathioprine, and procarbazine; antibiotics, including but not limited to doxorubicin, bleomycin, dactinomycin, daunorubicin, mycomycin, mitomycin, sarcomycin C and daunomycin; and other chemotherapeutic drugs, including but not limited to anti-tumor antibodies, dacarbazine, azacytidine, amsacang, melphalan, ifosfamide, and mitoxantrone.

In some embodiments, chemotherapeutic drugs that can be used in combination with immune cells of the invention include, but are not limited to, anti-angiogenic agents, including anti-VEGF antibodies (including humanized and chimeric antibodies, anti-VEGF aptamers, and antisense oligonucleotides) and other angiogenesis inhibitors, such as angiostatin, endostatin, interferon, retinoic acid, and tissue inhibitors of metalloproteinases-1 and-2.

### Combination Kit

The invention also provides a combination kit comprising the immune cells of the invention. The kit can be used to treat or prevent cancer, pathogen infection, immune disorders, or allotransplantation. In one embodiment, the kit may include a therapeutic or prophylactic composition comprising an effective amount of immune cells of one or more unit dosage forms.

In some embodiments, the kit includes a sterile container that may comprise a therapeutic or prophylactic composition.

In some cases, the kit may include about 1×10⁴ cells to about 1×10⁶ cells. In some cases, the kit may include at least about 1×10⁵ cells, at least about 1×10⁶ cells, at least about 1 × 10⁷ cells, at least about 4×10⁷ cells, at least about 5×10⁷ cells, at least about 6×10⁷ cells, at least about 6×10⁷ cells, 8×10⁷ cells, at least about 9×10⁷ cells, at least about 1×10⁸ cells, at least about 2×10⁸ cells, at least about 3×10⁸ cells, at least about 4×10⁸ cells, at least about 5×10⁸ cells, at least about 6×10⁸ cells, at least about 6×10⁸ cells, at least about 8×10⁸ cells, at least about 9×10⁸ cells, at least about 1×10⁹ cells, at least about 2×10⁹ cells, at least about 3×10⁹ cells, at least about 4×10⁹ cells, at least about 5×10⁹ cells, at least about 6×10⁹ cells, at least about 8×10⁹ cells, at least about 9×10⁹ cells, at least about 1×10¹⁰ cells, at least about 2×10¹⁰ cells, at least about 3×10¹⁰ cells, at least about 4×10¹⁰ cells, at least about 5×10¹⁰ cells, at least about 6×10¹⁰ cells, at least about 9×10¹⁰ cells, at least about 9×10¹⁰ cells, at least about 1×10¹¹ cells, at least about 2×10¹¹ cells, at least about 3×10¹¹ cells, at least about 4×10¹¹ cells, at least about 5×10¹¹ cells, at least about 8×10¹¹ cells, at least about 9×10¹¹ cells, or at least about 1×10¹² cells. For example, approximately 5×10¹⁰ cells can be comprised in the kit.

### Example 1: Construction of lentiviral plasmid of chimeric antigen receptor protein encoded by nucleic acid and virus packaging

Table 1 below shows the connection sequence of the parts of the chimeric antigen receptor of the Examples of the present invention.

**Table 1. Connection sequence of various parts of chimeric antigen receptor**

| Name | Connection sequence of various parts of chimeric antigen receptor |
|---|---|
| antiGPC3-synNotch-GAL4VP64 | PGK promoter (SEQ ID NO: 18) - signal peptide (SEQ ID NO: 1) - myc tag (SEQ ID NO: 19)- GPC3 scfv (SEQ ID NO: 2)-notch core (SEQ ID NO: 3) - GAL4VP64 (SEQ ID NO: 4) |
| GAL4UAS-IL12-PG K-mcherry | GAL4UAS (SEQ ID NO: 5) - CMV minimal promoter (SEQ ID NO: 6) - IL12 - PGK promoter - mcherry (SEQ ID NO: 20) |

| | |
|---|---|
| 1. Amplification of nucleic acid fragment of antiGPC3-synNotch-GAL4VP64 1) Amplification of GPC3 scFv sequence | |

The nucleic acid sequence of antiGPC3 scFv (SEQ ID NO: 2) was obtained by conventional PCR method.

PHR-PGK-antiCD19-synNotch-GAL4VP64 (purchased by addgene) was used as a template to obtain other nucleic acid sequences by PCR. The signal sequence (SEQ ID NO: 1) was PCR-amplified with a primer pair [upstream primer: 5'-tctcacgcgtcaagtggagc-3' (SEQ ID NO: 14), downstream primer: 5'-tctgcaccagctgcacctcgaggtcctcttcagag-3' (SEQ ID NO: 15)]; and the synNotch-GAL4VP64 sequence was PCR-amplified with a primer pair [upstream primer: 5'-atcctggactacagcttcacaggtggcgc-3' (SEQ ID NO: 16), downstream primer: 5'-agagccggcagcaggccgcgggaag -3' (SEQ ID NO: 17)].

### 2. Slicing of nucleic acid fragment of antiGPC3-synNotch-GAL4VP64

The antiGPC3 scfv nucleic acid fragment, an equimolar signal sequence nucleic acid fragment and equimolar synNotch-GAL4VP64 nucleic acid fragment were subjected to three-segment splicing and PCR. The splicing conditions were: pre-denaturation: 94°C, 4min; denaturation: 94°C, 40s; annealing : 42°C, 40s; extension: 68°C, 3min 20s, 7 cycles, then total extension 68°C, 10 min. And then DNA polymerase, forward primer and reverse primer were supplemented, and PCR amplification was performed for 30 cycles, wherein the amplification conditions were: pre-denaturation: 94°C, 4 min; denaturation: 94°C, 40s; annealing: 60°C, 40s; extension: 68°C, 3min 20s, for 30 cycles, then a total extension 68°C, 10 min. The amplified fragment was antiGPC3-synNotch-GAL4VP64.

### 3. Amplification of nucleic acid fragment of GAL4UAS-IL12-PGK-mcherry

Firstly, pHR-GAL4UAS-tBFP-PGK-mcherry (purchased from addgene) was used as a template to insert an MluI restriction site between tBFP and PGK-mcherry, and then this plasmid was used as a template for further construction.

### 1) Amplification of IL12 sequence

The IL12 sequence is obtained by PCR amplification (wherein, the nucleic acid sequence of IL12 P40 is shown in SEQ ID NO: 7 and the nucleic acid sequence of IL12 P35 is shown in SEQ ID NO: 8).

### 2) Nucleic acid sequence of other parts

The nucleic acid sequences of other parts were obtained by PCR using pHR-GAL4UAS-tBFP-PGK-mcherry as a template. The GAL4UAS sequence was PCR-amplified with a primer pair [upstream primer: 5'-agatcgcgatgggaaaaaattc-3' (SEQ ID NO: 28), downstream primer: 5'-tgctgaacttcactctcatgatccaacgaatgtcgag-3' (SEQ ID NO: 29)].

### 4. Slicing of nucleic acid fragment of GAL4UAS-IL12

The above obtained IL12 nucleic acid fragment and an equimolar GAL4UAS nucleic acid fragment were subjected to two-segment splicing and PCR. The splicing conditions were: pre-denaturation: 94°C, 4 min; denaturation: 94°C, 40 s; annealing : 42°C, 40s; extension: 68°C, 3 min 20s, 7 cycles, then total extension 68°C, 10 min. And then DNA polymerase, forward primer and reverse primer were supplemented, and PCR amplification was performed for 30 cycles, wherein the amplification conditions were: pre-denaturation: 94°C, 4 min; denaturation: 94°C, 40s; annealing: 60°C, 40s; extension: 68°C, 3min 20s, for 30 cycles, then a total extension 68°C, 10 min. The amplified fragment was GAL4UAS-IL12.

### 5. Construction of Lentiviral Plasmid Vector

The vector system used in the lentiviral plasmid vector belongs to the third-generation of self-inactivating lentiviral vector system. There are three plasmids in the system, namely the encoding protein Gag/Pol, the packaging plasmid psPAX2 (purchased from addgene) encoding Rev protein; and envelope plasmid PMD2.G (purchased from addgene) encoding VSV-G protein.

The target gene antiGPC3-synNotch-GAL4VP64 obtained in the above steps 1 and 2 was digested with MluI and SacII restriction enzymes, and then connected to the pHRLSIN vector (purchased from addgene) which was subjected to the same double digestion; and the target gene GAL4UAS-IL12 was digested with NruI and MluI restriction enzymes, and then connected to the pHRLSIN vector which was subjected to the same double digestion and addition of MluI. The successfully constructed vector was sequenced as being correct, so that can be used for lentivirus packaging.

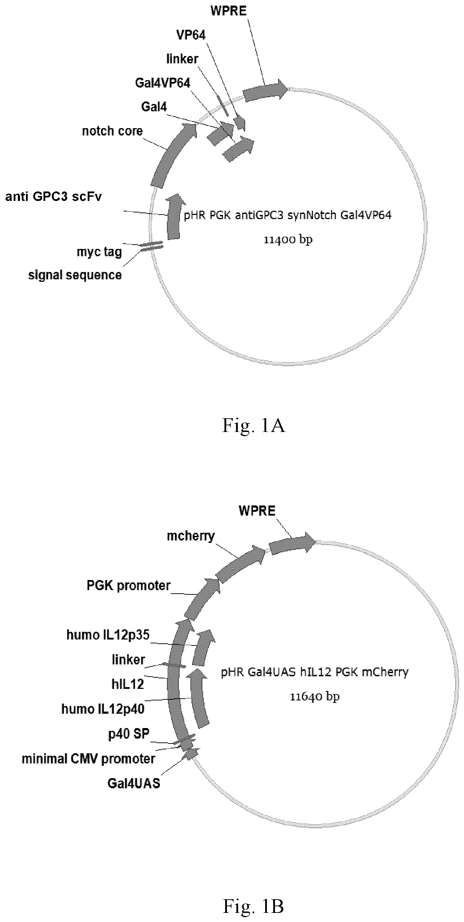

The obtained vectors containing the target fragments are shown as follows (see Figure 1A and Figure 1B for functional elements and connection relationships):
pHRLSIN- antiGPC3-synNotch-GAL4VP64 (i.e., pHR-antiGPC3-synNotch -GAL4VP64, wherein the sequence of antiGPC3-synNotch-GAL4VP64 was shown im SEQ ID NO: 21);
pHRLSIN-GAL4UAS-IL12-PGK-mcherry (i.e., pHR-GAL4UAS-IL12-PGK-mcherry, the sequence of which was shown in SEQ ID NO: 22).

### 6. Transfection of 293T cells with plasmids to package lentivirus

1) 293T cells cultured to the 6^{th} ∼ 10^{th} passages were inoculated into a 10 cm petri dish at a density of 5×10⁶, and cultured overnight at 37°C and 5% CO₂ to prepare for transfection, and the medium was a medium containing 10% fetal bovine serum DMEM;
2) 10 µg of target gene plasmids pHRLSIN-antiGPC3-synNotch-GAL4VP64, pHRLSIN-GAL4UAS-IL12-PGK-mcherry were added with 3 µg of the envelope plasmid pMD2.G and 7.5 µg of the packaging plasmid pAX2 were added into 800 µl of blank DMEM medium, respectively and mixed well;
3) 60 µg of polyetherimide (PEI) (1 µg/µl) was dissolved in 800 µl serum-free DMEM medium, mixed gently (or vortexed at 1000 rpm for 5 seconds) and incubated for 5 min;
4) formation of transfection complex: the plasmid mixture was added into the PEI mixture, vortexed or gently mixed immediately upon addition, and incubated for 20 min at room temperature;
5) 1.6 ml of transfection complex was dropped into a 10 cm petri dish containing 11 ml of DMEM medium (it is not necessary to change the medium);
6) After 4-5h, the medium was change with DMEM medium containing 10% FBS for the transfected 293T cells;
7) After 72 hours of transfection, the virus supernatant was filtered and collected with a 0.45 µm filter;
8) The virus was concentrated at 5×PEG-8000 NaCl, and 7.5 ml of mother liquid was added per 30 ml of initial solution. The obtained liquid was mixed every 20-30min for a total of 3-5 times, and placed overnight at 4°C;
9) The next day, the obtained liquid was subjected to centrifuge at 4000 rpm for 60 min at 4°C, the supernatant was discarded, and the precipitate obtained from the centrifugation was resuspended in a dissolving solution at 1/10-1/50 of the original volume and stored in aliquots (100 µl/tube) at -80°C to obtain lentivirus 1.

### 7. Determination of Lentivirus Titer

1) 293T cells were inoculated in a 12-well culture plate at 1×10⁵ cells, 1 ml/well; a polybrene solution, the initial concentration of which was 10 µg/µl, was added at 0.6 µl/ml and the final concentration was 6 µg/ml; the cells were incubated at 37°C, 5% CO₂ for 1 hour, and the culture medium was DMEM containing 10% fetal bovine serum;
2) 10 µl/well of virus concentrate was added for 5-fold dilution, 3 gradients, and incubated at 37°C and 5% CO₂;
3) After 72 hours, 293T cells were trypsinized (30s), and 1 ml DMEM (10% FBS) was added to terminate the digestion. The cell suspension was transferred to a 2 ml centrifuge tube (half), centrifuged at 5000 rpm for 5 min, the supernatant was discarded, and the precipitate washed twice with PBS (2% N-bromosuccinimide (NBS));
4) Cells in the pHRLSIN-antiGPC3-synNotch-GAL4VP64 virus control group were washed twice with PBS (2% NBS) and resuspended as a control; the cells in the test group were described as above, the cells in the test group + 50 µl 1: 50 diluted Alexa(R)-647-labeled anti-myc antibody were incubated on ice for 45 min, 5000 rpm/min and centrifuged for 5min, and the supernatant was discarded; the above procedure was repeated twice; 500 µl PBS (2% NBS) was added, transferred to the flow tube, and Alexa (R)-647 channel was detected with a flow cytometer.
5) For PHRLSIN-GAL4UAS-IL12-PGK-mcherry virus, PE-CF594 channel was directly detected with a flow cytometer;
6) The number of cells with a positive rate of 5-20% was appropriate, and the titer was calculated as: the titer (U/mL) = the number of cells × positive rate/virus volume. The virus titer measured after concentration was about 1×10⁸ U/ml for pHRLSIN-antiGPC3-synnotch-GAL4VP64; and 3×10⁸ U/ml for pHRLSIN-GAL4UAS-IL12-PGK-mcherry.

### Example 2: Infection of NK92 cells with recombinant lentivirus

NK92 cells were infected with the lentivirus 1 prepared in Example 1 to obtain GPC3-SYN-IL12-NK92 cells. The specific operations are listed as follows:
1) On the day before infection, a 24-well plate was coated with recombinant human fibronectin (Retronectin), and 380 µl of 5 µg/ml recombinant human fibronectin solution (PBS) was added to each well, and incubated overnight at 4°C;
2) At the time of infection, the recombinant human fibronectin solution (PBS) in the 24-well plate was discarded, and the plate was washed twice with 1 ml PBS. The above recombinant lentivirus was used in infection at MOI=30, polybrene at a final concentration of 10 µg/ml was added to improve the infection efficiency. The number of cells per well was 5× 10⁵, the volume of the culture medium was 500 µl, and the cells were centrifuged at 32°C, 1800 g for 90min, and then transferred to an incubator;
3) The infected cells were passaged at a density of 5×10⁵ /ml every other day.

### Example 3: Identification of GPC3-SYN-IL12-NK92 cells

On the 7^{th} day of culture, NK92 cells infected with lentivirus were tested for the expression of different receptors by flow cytometry. Since a Myc tag is present at the N-terminal of antiGPC3, the detected Myc expression means a positive cell successfully infected with pHRLSIN-antiGPC3-synNotch-GAL4VP64, the detected mcherry expression means a positive cell successfully infected with pHRLSIN-GAL4UAS-IL12-PGK-mcherry, and the simultaneous expression of Myc and mcherry means a positive cells GPC3-SYN-IL12-NK92 with successful double infection.
1) 1×10⁶ cells were taken from different infected NK92 cells respectively, divided into 2 ml centrifuge tubes, and centrifuged at 4°C, 5000rpm for 5 min, the supernatant was discarded, and the precipitate was washed twice with PBS;
2) The cells in the control group for detecting myc expression were directly washed twice with PBS (2% NBS) and resuspended as a control; the cells in the detection group cells are described as above, and the cells in the detection group + 50 µl of 1: 50 diluted Alexa(R)-647 labeled anti-myc antibody were incubated for 45 minutes on ice;
3) 2 ml of PBS (2% NBS) was added to resuspend the cells, the cells were centrifuged at 5000 rpm/min, 4°C for 5 min and the supernatant was discarded; and the procedure was repeated twice;
4) 500 µl of PBS (2% NBS) was added and transferred to a flow tube. Alexa(R)-647 and PE-CF594 channels were detected by a Flow cytometer to determine the proportion of positive NK92 cells.

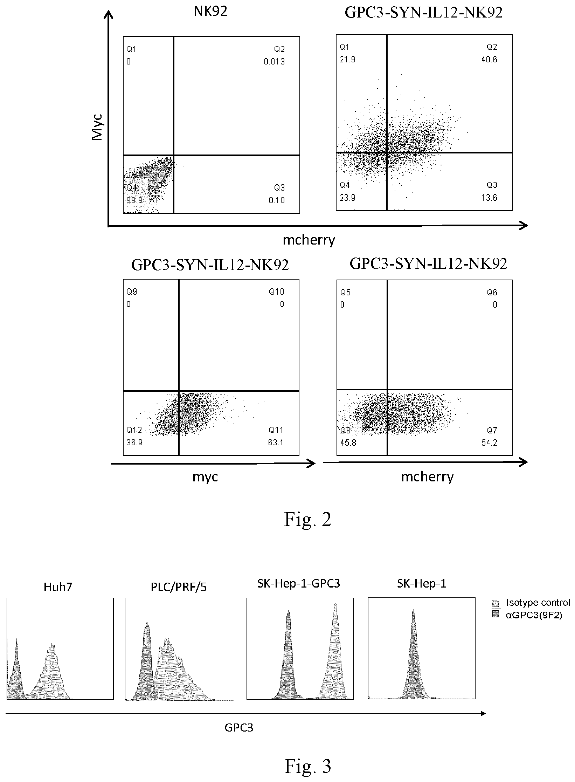

The results are shown in Figure 2. The double positive rate for Myc and Mcherry tags in GPC3-SYN-IL12-NK92 cells was 40.6%, in which the positive rate for Myc was 63.1% and the positive rate for Mcherry was 54.2%.

GPC3-SYN-IL12-NK92 cells were subcultured and counted every other day at a cell density of 5×10⁵/ml. On the 11^{th} day of culture, there was about 20-40 times expansion, indicating that there were a certain number of *in vitro* expansion for the double-infected NK92 cells, thereby guaranteeing the subsequent *in vitro* toxicity test and *in vivo* test.

### Example 4: Regulated expression of IL12 in GPC3-SYN-IL12-NK92 cells

1) The materials used for regulating expression are listed as follows: liver cancer cell line (Huh7 (high expression of GPC3), PLC/PFR/5 (low expression of GPC3), SK-Hep-1 (no expression of GPC3), SK-Hep-1-GPC3 (overexpression of GPC3)) as a target cell antigen for stimulation. The expressions of GPC3 in each target cells are shown in Figure 3. Effector cells are NK92 cells expressing chimeric antigen receptors cultured for 12 days *in vitro*;
2) The effector to target ratio (effector: target) is 1: 1, the cells were spreaded in a 12-well plate at 2×10⁵/well; and centrifuged at 400 g for 1 min to increase mutual contact;
3) Each experimental group and each control group are listed as follows:
   experimental group: each target cell + GPC3-SYN-IL12-NK92;
   control group: each target cell + NK92
4) The supernatant was collected after co-culture at 37°C and 5% CO₂ for 24 hours, and the expression of IL12 was detected by Elisa kit.

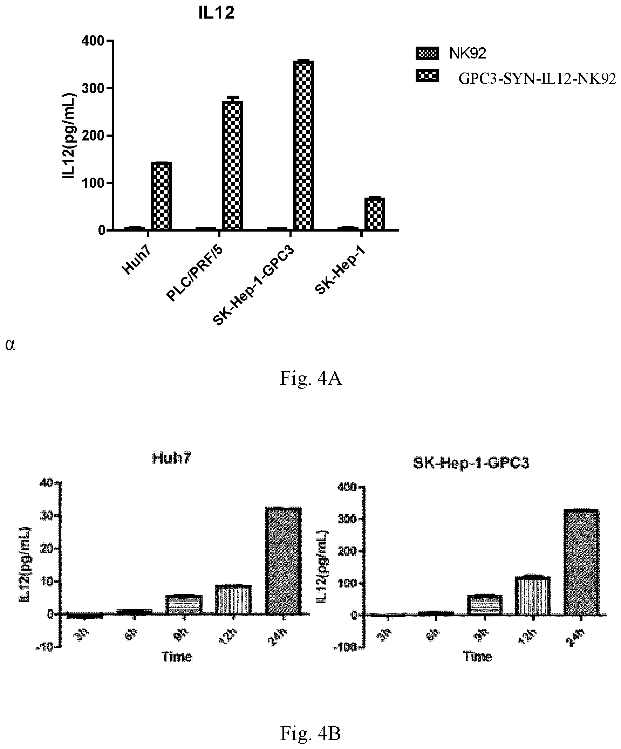

From the results of Elisa in Figure 4A, it can be seen that, after co-incubated with cell lines Huh7, PLC (high or medium expression of GPC3) and SK-Hep-1 (overexpression of GPC3) respectively, the secretion of IL12 from GPC3-SYN-IL12-NK92 cells of the present invention was significantly increased, but there was only a little basic expression of IL12 in SK-Hep-1 cells not expressing GPC3, indicating that the secretion of IL12 from GPC3-SYN-IL12-NK92 cells depends on the stimulation and regulation of the target antigen.

During the co-incubation of GPC3-Syn-IL12 NK92 cells with Huh7 cells and SK-Hep-1-GPC3, the secretion of IL12 at different time points is shown in Figure 4B, which shows that the secretion of IL12 is greatly increased over time.

### Example 5: Construction of CAR-T cells

As an example, CAR-T cells targeting GPC3 were constructed in this example, the CAR of which has the amino acid sequence as shown in SEQ ID NO: 23. It should be understood that CARs of other generations also have similar effects, for example, GPC-BBZ, GPC-28BBZ, GPC3- z as shown in SEQ ID NO: 24, SEQ ID NO: 25, SEQ ID NO: 24 and SEQ ID NO: 26.

pRRLSIN-cPPT.EF-1α was used as a vecto to construct a lentiviral plasmid expressing the second-generation of chimeric antigen receptor of the humanized antibody huGPC3, PRRLSIN-cPPT.EF-1α-huGPC3-28Z. The sequence of HuGPC3-28Z consists of CD8α signal peptide (SEQ ID NO: 9), huGPC3 scFV (SEQ ID NO: 2), CD8 hinge (SEQ ID NO: 10), CD28 transmembrane region (SEQ ID NO: 11), intracellular signaling domain (SEQ ID NO: 12) and intracellular segment of CD3, CD3ξ (SEQ ID NO: 13).

According to the operation of Example 1, 293T cells were transfect to package lentivirus, thereby obtaining lentivirus 2.

T cells were infected with Lentivirus 2 to obtain GPC3-28Z CAR-T cells.

### Example 6: In vitro experiments

CytoTox 96 non-radioactive cytotoxicity detection kit (Promega Company) was used according to the instructions of CytoTox 96 non-radioactive cytotoxicity detection kit.
1) Target cells: 50 µL of 2×10⁵/mL huh7 cells, PLC/PRF/5, SK-Hep-1-GPC3, SK-Hep-1 cells were inoculated into 96 well plates, respectively;
2) Effector cells: UTD, UTD+GPC3-Syn-IL12, GPC3-28Z, and GPC3-28Z+ GPC3-Syn-IL12 cells were added at an effector target ratio of 3: 1, 1: 1 or 1: 3.

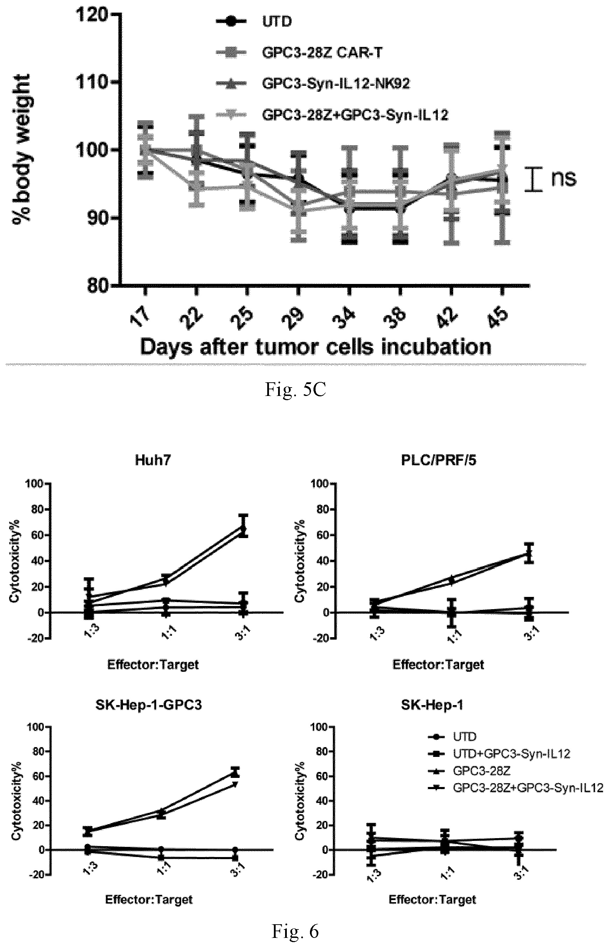

The effector cells and target cells were co-incubated for 12 hours. The experiment results are shown in Figure 6.

### Example 7: In vivo tumor suppression experiment

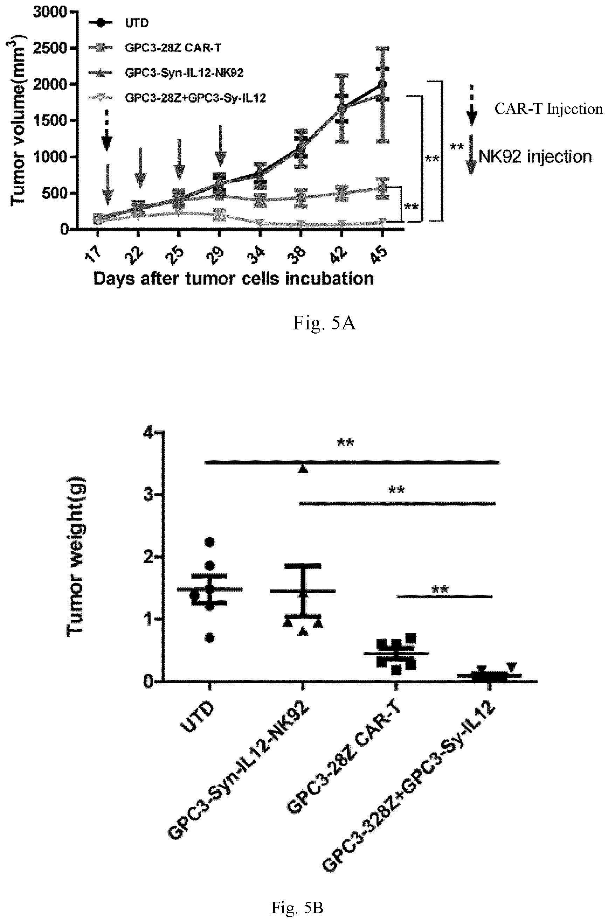

a. NSG mice were inoculated with Huh7 transplanted tumor
   Huh7 cells were subcutaneously inoculated, 2×106 per mouse.
b. Preparation of UTD, GPC3-28Z CAR-T cells and double-infected GPC3-SYN-IL12-NK92
   1) On day d0, 3×10⁷ PBMC cells were taken, magnetic beads were added at CD3-CD28 antibody coated magnetic beads: cells = 2: 1, medium was AIM-V (2% AB serum), and the total volume was 5ml. And IL-2 was added with a final concentration of 500 IU/ml;
   2) On day 1, a 24-well cell culture plate was coated with recombinant human fibronectin: the concentration of recombinant human fibronectin was 5 µg/ml at 380 µl/well, and incubated overnight at 4°C;
   3) On day d2, the activated cells were taken for virus infection. The number of PBMC infected by each virus was 3×10⁶ and MOI = 10. The cells and virus liquid were transferred to a 24-well plate coated with recombinant human fibronectin, and the infection conditions were 32°C and centrifugation at 1800 rpm for 40 min;
   4) On day 3, the infected PBMC cells were centrifuged at low speed (500 rpm, 10 min) to remove dead cells and then the medium was changed;
   5) On day 5, the CD3-CD28 antibody-coated magnetic beads were removed with magnetic poles, and incubated for up to 12 days.
c. Combined adoptive immunotherapy of GPC3-28Z CAR-T cells and GPC3-SYN-IL12-NK92 cells were performed on Huh7 transplanted tumors.
   On day 18, the volume of the transplanted tumor of Huh7 in NSG mice was measured, which was about 150 mm³. Mice were divided into 4 groups, including: UTD control group, GPC3-28Z CAR-T single treatment group, GPC3-SYN-IL12-NK92 single treatment group, and GPC3-28Z CAR-T + GPC3-SYN-IL12-NK92 combined treatment groups, 6 mice in each group; and adoptive immunotherapy was performed on the mice in each group. 200 µl of cells were injected through the tail vein.
   (a) UTD group (T cell group without being transfected with vector): 1×10⁶/UTD.
   (b) GPC3-28Z CAR-T cell group: 1×10⁶/positive cells.
   (c) GPC3-SYN-IL12-NK92 group: 1×10⁶/positive cells.
   (d) GPC3-28Z CAR-T + GPC3-SYN-IL12-NK92 combined treatment group: 1×10⁶/positive cells, and then every 3-4 days, GPC3-SYN-IL12-NK92 cells were administered again, for a total of four times, each time 1×10⁶/positive cell.
d. the volume of Huh7 transplanted tumor was measured every 3-4 days, the change of tumor volume in each group of mice was recorded, and tumor volume was calculated according to: (length × width²) / 2. The results are shown in Figure 5A. It can be seen that the GPC3-28Z CAR-T + GPC3-SYN-IL12-NK92 combination treatment group can significantly inhibit the growth of the tumor.

On Day 45, the mice were killed by cervical dislocation, the subcutaneous tumor in the mouse was peeled off and weighed. The results are shown in Figure 5B. Compared with the control group, the weight of the tumor of the mice in the GPC3-28Z CAR-T + GPC3-SYN-IL12-NK92 combination treatment group was lighter.

When recording the changes in the tumor volume of mice in each group, the weight changes of the mice are shown in Figure 5C, and there is no statistical difference in the volume changes of the mice in each treatment group. The statistical method of the data is ±SEM, *P <0.05, **P <0.01, ***P <0.001, ****P <0.0001, 2 way ANOVA.

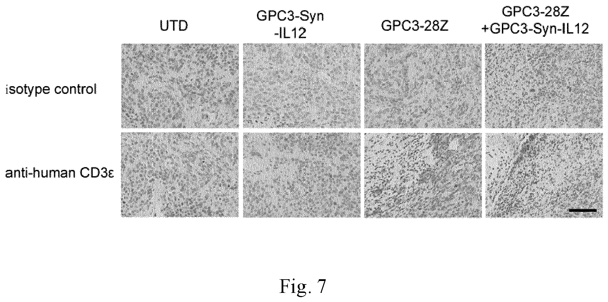

The tumor tissues of mice were prepared into tissue sections by using conventional tissue section preparation methods. The tissue sections were immunohistochemically stained, and anti-human CD3 antibody (Thermo Scientific) was used to detect human CAR-T cells in the tumor tissue sections. The results are shown in Figure 7, showing that there are more CAR-T cell infiltrations in GPC3-28Z CAR-T + GPC3-SYN-IL12-NK92 combination treatment group. The above results indicate that there is a synergistic effect between the two cells in the combination treatment group.

Conclusion: In *in vitro* experiments, compared with the control group, GPC3-28Z + GPC3-Syn-IL12 cells exhibit no significant difference in killing tumor cells. In *in vivo* experiments, compared with the control group, in GPC3-28Z CAR-T + GPC3-SYN-IL12-NK92 combination treatment group, the tumor growth of mice was significantly inhibited.

The description of the above embodiments is only helpful for understanding the core idea of the present invention. It should be pointed out that a skilled person in the art can make improvements and modifications to the solid powder composition of the present invention and the preparation method thereof without departing from the principle of the present invention, and these improvements and modifications also fall within the scope of the claims of the present invention.

## Claims

1. A pair of plasmid vectors including a first vector and a second vector, wherein the first vector comprises a Notch core and further comprises encoding nucleic acid molecules for other extracellular segments and intracellular segments, and preferably, the intracellular segment comprises transcriptional activator; and the second vector comprises a nucleic acid molecule that specifically recognizes and binds to the intracellular segment in the first vector and a nucleic acid molecule encoding IL12.

2. The pair of plasmid vectors of claim 1, wherein the Notch core of the first vector has the nucleic acid sequence as shown in SEQ ID NO: 3; and preferably, the intracellular segment of the first vector is GAL4VP64, and the second vector comprises GAL4UAS.

3. The pair of plasmid vectors of claim 1, wherein the extracellular segment comprises an antigen or an antibody or fragment thereof of the antigen, a receptor or a ligand of the receptor.

4. The pair of plasmid vectors of claim 1, wherein the extracellular segment of the first vector can specifically recognize a tumor antigen; preferably, the tumor antigen is a solid tumor antigen; and the tumor antigen is one or more selected from the group consisting of prostate specific membrane antigen (PSMA), carcinoembryonic antigen (CEA), IL13Ralpha, HER-2, CD19, NY-ESO-1, HIV-1 Gag, Lewis Y, MART-1 , Gp100, tyrosinase, WT-I, hTERT, mesothelin, EGFR, EGFRvIII, GPC3, EphA2, HER3, EpCAM, MUC1, MUC16, CLDN18.2, folate receptor, CLDN6, CD30, CD138, ASGPR1 CDH16, GD2, 5T4, 8H9, αvβ6 integrin, B cell maturation antigen (BCMA), B7-H3, B7-H6, CAIX, CA9, CD20, CD22, kappa light chain, CD33, CD38, CD44 , CD44v6, CD44v7/8, CD70, CD123, CD171, CSPG4, EGP2, EGP40, ERBB3, ERBB4, ErbB3/4, FAP, FAR, FBP, embryonic AchR, GD2, GD3, HLA-AI MAGE A1, MAGE3, HLA -A2, IL11Ra, KDR, Lambda, MCSP, NCAM, NKG2D ligand, PRAME, PSCA, PSC1, ROR1, Sp17, SURVIVIN, TAG72, TEM1, TEM8, VEGRR2, HMW-MAA, VEGF receptor, and/or fibronectin, cytotactin or carcinoembryogenic variants of tumor necrosis region; and more preferably, the tumor antigen is is GPC3 or CLDN18.2.

5. The pair of plasmid vectors of claim 1, wherein the extracellular segment comprise scFv; preferably, anti-GPC3 scFv.

6. The pair of plasmid vectors of claim 1, wherein the first vector also comprised a label, for example, C-myc, FLAG and/or HA.

7. The pair of plasmid vectors of claim 1, wherein the second vector further comprises a reporter, for example GFP, BFP and / or mcherry.

8. The pair of plasmid vectors of claim 1, wherein the first vector sequentially comprises PGK promoter - signal sequence - myc tag - anti-GPC3 scFv - notch core - GAL4 - linker - VP64, and the second vector sequentially comprises GAL4UAS - CMV minimal promoter - IL12 - PGK promoter - mcherry.

9. The pair of plasmid vectors of claim 1, wherein the open reading frame of the first vector comprises a nucleotide sequence as shown in SEQ ID NO: 21, and the open reading frame of the second vector comprises a nucleotide as shown in SEQ ID NO: 22.

10. The pair of plasmid vectors of any one of claims 1-9, wherein the first vector or the second vector further comprises respective functional variant, and the nucleotide sequence of the variant has at least 50%, preferably 70%, 75%, 80% , 85% or 90%, more preferably 95%, 96%, 97%, 98%, 99% identity with the nucleotide sequence of the first vector or the second vector, respectively; and preferably, the anti-GPC3 scFv has at least 90% identity with the nucleotide sequence as shown in SEQ ID NO: 2.

11. An genetically modified immune cell, wherein the cell is transfected with the pair of plasmid vectors of any one of claims 1-10.

12. The immune cell of claim 11, wherein the immune cell is a T cell or NK cell; and preferably, the immune cell is a NK cell.

13. The immune cell of claim 12, wherein the NK cell is derived from blood or a cell line; preferably, from a cell line; and more preferably, the NK cell from a cell line is NK92 cell line.

14. Uses of the pair of the plasmid vectors of any one of the claims 1-10 or the immune cell of any one of claims 11-13 in the preparation of a medicament for local secretion of IL12 or regulating expression of IL12.

15. Uses of the pair of the plasmid vectors of any one of the claims 1-10 or the immune cell of any one of claims 11-13 in the preparation of a medicament for enhancing killing effects of CAR-T cells.

16. The use of claim 15, wherein the tumor antigen specifically recognized by the extracellular segment of the first vector of the immune cell is the same as the tumor antigen recognized by the CAR-T cell.

17. The use of claim 15, wherein both of the tumor antigen specifically recognized by the extracellular segment of the first vector of the immune cell and the tumor antigen recognized by the CAR-T cell are expressed on the same tumor.

18. The use of claim 16, wherein both of the tumor antigen specifically recognized by the extracellular segment of the first vector of the immune cell and the tumor antigen recognized by the CAR-T cells are GPC3.

19. The use of claim 17, wherein both of the tumor antigen specifically recognized by the extracellular segment of the first vector of the immune cell and the tumor antigen recognized by the CAR-T cells are expressed in liver cancer.

20. The use of claim 17, wherein the first vector of the immune cell comprises the sequence as shown in SEQ ID NO: 21, and the second vector comprises the sequence as shown in SEQ ID NO: 22; and the CAR of the CAR-T cell has the sequence as shown in SEQ ID NO: 23, 24 or 25.

21. The use of any one of claims 14-20, wherein the administrated ratio of the immune cell to CAR-T cell is 1: 1 ∼ 4: 1.

22. The use of any one of claims 14-21, wherein the immune cells can be administrated in multiple times within one administration cycle.

23. A composition comprising the immune cell of any one of claims 11-13 and a CAR-T cell.

24. The composition of claim 23, wherein the tumor antigen specifically recognized by the extracellular segment of the first vector of the immune cell is the same as the tumor antigen recognized by the CAR-T cell.

25. The composition of claim 23, wherein both of the tumor antigen specifically recognized by the extracellular segment of the first vector of the immune cell and the tumor antigen recognized by the CAR-T cell are expressed on the same tumor.

26. The composition of claim 24, wherein both of the tumor antigen specifically recognized by the extracellular segment of the first vector of the immune cell and the tumor antigen recognized by the CAR-T cell are GPC3.

27. The composition of claim 25, wherein both of the tumor antigen specifically recognized by the extracellular segment of the first vector of the immune cell and the tumor antigen recognized by the CAR-T cell are expressed in liver cancer.

28. The composition of claim 23, wherein the first vector of the immune cell comprises the sequence as shown in SEQ ID NO: 21, the second vector comprises the sequence as shown in SEQ ID NO: 22, and
the CAR of the CAR-T cell comprises the sequence as shown in SEQ ID NO: 23, 24 or 25.

29. The composition of any one of claims 23-28, wherein the administrated ratio of the immune cell to CAR-T cell is 1: 1 ∼ 4: 1.

30. A combination of kits, including kit A comprising the immune cell of any one of claims 11-13 and kit B comprising a CAR-T cell.

31. The combination of claim 30, wherein the tumor antigen specifically recognized by the extracellular domain of the first vector of the immune cell is the same as the tumor antigen recognized by the CAR-T cell.

32. The combination of claim 30, wherein both of the tumor antigen specifically recognized by the extracellular domain of the first vector of the immune cell and the tumor antigen recognized by the CAR-T cell are expressed on the same tumor.

33. The combination of claim 31, wherein both of the tumor antigen specifically recognized by the extracellular domain of the first vector of the immune cell and the tumor antigen recognized by the CAR-T cell are GPC3.

34. The combination of claim 32, wherein both of the tumor antigen specifically recognized by the extracellular domain of the first vector of the immune cell and the tumor antigen recognized by the CAR-T cell are expressed in liver cancer.

35. The combination of claim 30, wherein the first vector of the immune cell comprises the sequence as shown in SEQ ID NO: 21, the second vector comprises the sequence as shown in SEQ ID NO: 22, and the CAR of the CAR-T cell comprises the sequence as shown in SEQ ID NO: 23, 24 or 25.
